(19) Europäisches Patentamt
European Patent Office
Office européen des brevets

(11) **EP 4 512 908 A2**

(12) **EUROPEAN PATENT APPLICATION**

(43) Date of publication:
26.02.2025 Bulletin 2025/09

(21) Application number: 24208177.6

(22) Date of filing: 19.05.2015

(51) International Patent Classification (IPC):
***C12Q 1/6858*** (2018.01)

(52) Cooperative Patent Classification (CPC):
(C-Sets available)
**C12Q 1/686; C12Q 1/6858** (Cont.)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 19.05.2014 US 201462000114 P

(62) Document number(s) of the earlier application(s) in
accordance with Art. 76 EPC:
**21150302.4 / 3 901 278**
**15796383.6 / 3 146 080**

(71) Applicant: **William Marsh Rice University**
**Houston, TX 77005 (US)**

(72) Inventors:
• **ZHANG, David Yu**
**Houston, 77030 (US)**
• **WU, Ruojia**
**Houston, 77054 (US)**
• **WANG, Juexiao**
**Houston, 77054 (US)**

(74) Representative: **Potter Clarkson**
**Chapel Quarter**
**Mount Street**
**Nottingham NG1 6HQ (GB)**

Remarks:
•The complete document including Reference Table(s) and the Sequence Listing(s) can be downloaded from the EPO website
•This application was filed on 22-10-2024 as a divisional application to the application mentioned under INID code 62.
•Claims filed after the date of filing of the application / after the date of recipt of the divisional application (Rule 68(4) EPC).

(54) **ALLELE-SPECIFIC AMPLIFICATION USING A COMPOSITION OF OVERLAPPING NON-ALLELE-SPECIFIC PRIMER AND ALLELE-SPECIFIC BLOCKER OLIGONUCLEOTIDES**

(57) The present invention provides an oligonucleotide composition including a blocker and a first primer oligonucleotide. The blocker oligonucleotide includes a first sequence having a target-neutral subsequence and a blocker variable subsequence. The non-target specific subsequence is flanked on its 3' and 5' ends by the target-neutral subsequence and is continuous with the target-neutral subsequence. The first primer oligonucleotide is sufficient to induce enzymatic extension; herein the first primer oligonucleotide includes a second sequence. The second sequence overlaps with the 5' end of the target-neutral subsequence by at least 5 nucleotides; herein the second sequence includes an overlapping subsequence and a non-overlapping subsequence. The second sequence does not include the non-target specific subsequence.

FIG. 2

EP 4 512 908 A2

(52) Cooperative Patent Classification (CPC): (Cont.)

C-Sets
**C12Q 1/6858, C12Q 2525/161, C12Q 2527/107,
C12Q 2537/143, C12Q 2537/163;
C12Q 1/686, C12Q 2525/161, C12Q 2527/107,
C12Q 2537/143, C12Q 2537/163**

**Description**

**CROSS-REFERENCE TO RELATED APPLICATIONS**

The present application claims priority to U.S. Provisional Application No. 62/000,114 filed on May 19, 2014, the disclosure of which is incorporated herein by reference in its entirety.

**SEQUENCE LISTING**

The sequence listing is filed with the application in electronic format only and is incorporated by reference herein. The sequence listing text file "14-21013-WO(260947.00251)_SL.txt" was created on May 18, 2015, and is 13,144 bytes in size.

**BACKGROUND**

Small differences in DNA and RNA sequence can lead to big differences in overall physical health and wellness of organisms including human beings. For example, a single-base change in a bacterial genome can lead to antibiotic resistance, and a single-base change in a human genome can lead to cancer progression. With the maturation of the genomics field and the accompanying discovery of many nucleic acid biomarker sequences and molecules, there is a strong demand from the biotechnology industry to develop reliable, robust, inexpensive, and precise nucleic acid assays that can discriminate single-base changes. In particular, many PCR-based assays have been developed that are allele-specific.

PCR-based approaches to the selective detection of rare mutations can be broadly classified into two families of approaches: (A) allele-specific primers, and (B) non-allele-specific primers with allele-specific blockers.

Examples of allele-specific primers include the amplification refractory mutation system (ARMS), allele-specific blocker PCR (ASB-PCR), and competitive allele specific TaqMan PCR (castPCR). Examples of (B) include PNA blocker PCR and co-amplification at lower denaturation temperature PCR (COLD-PCR).

Prior to the present invention, allele-specific primers have generally been superior (higher mutation sensitivity) at detecting known single base mutations, while non-allele-specific primers with allele-specific blockers are generally applied for the detection of multiple closely clustered mutations (hotspots) or unknown mutation sequences.

Allele-specific PCR primers such as ARMS, ASB-PCR, castPCR generally possess an allele-specific nucleotide at the 3'-most position of the primer. The allele-specific PCR primers employ the discrimination of the DNA polymerase enzyme to specifically extend properly paired bases, but are limited by the fact that, when an incorrect DNA polymerase extension event does occur, the rare allele nucleotide that was a part of the primer becomes incorporated in the template, and subsequently amplification cycles become non-specific. The allele-specific PCR primers are only specific up to the first incorrect amplification event. Allele-specific detection with non-allele-specific primers requires precise denaturation temperature control, and restricted analysis of smaller sequences. The allele-specific detection with non-allele-specific primers has low mutation sensitivity and is more vulnerable to polymerase-introduced errors.

Thus, a composition of non-allele-specific primer and allele-specific blocker oligonucleotides with increased allele-specific amplification and improved mutation sensitivity is needed. Therefore, the development of new approaches suitable for the detection of a small amount of target in a large excess of variant is a prerequisite for the diagnostic applications. Thus, the present disclosure provides a composition of non-allele-specific primer and allele-specific blocker oligonucleotides to provide a more accurate and an efficient tool for disease diagnostic applications such as, cancer diagnosis and like.

**SUMMARY**

The present invention relates to a composition of overlapping a non-allele-specific primer oligonucleotide and an allele-specific blocker oligonucleotide for allele-specific amplification.

The present invention provides an oligonucleotide composition including a blocker and a first primer oligonucleotide. The blocker oligonucleotide includes a first sequence having a target-neutral subsequence and a blocker variable subsequence. However, in some instances, the blocker oligonucleotide may not include the blocker variable subsequence if the target nucleic acid to be detected is for the detection of an insertion. The blocker variable subsequence is flanked on its 3' and 5' ends by the target-neutral subsequence and is continuous with the target-neutral subsequence. The first primer oligonucleotide is sufficient to induce enzymatic extension; herein the first primer oligonucleotide includes a second sequence. The second sequence overlaps with the 5' end of the target-neutral subsequence by at least 5 nucleotides such that the second sequence includes an overlapping subsequence and a non-overlapping subsequence. The second sequence does not include any sequence homologous with the blocker variable subsequence.

The present invention further relates to a method for amplification of a target sequence. The method includes obtaining a sample containing one or more copies of a first nucleic acid having a variant sequence and at least one copy of a second nucleic acid; herein the second nucleic acid have the target sequence. The target sequence and variant sequence each includes a homologous subsequence and a variable subsequence. The variable subsequence further includes at least one nucleotide. Furthermore, the variable subsequence of the target sequence is a target-specific subsequence and the variable subsequence of the variant sequence is a non-target specific subsequence.

After obtaining the sample, a blocker oligonucleotide is introduced to the sample. The blocker oligonucleotide includes a first sequence having a target-neutral subsequence and a blocker variable subsequence. The target-neutral subsequence is complementary to a portion of the homologous subsequence and the blocker variable subsequence is complementary to the non-target specific subsequence. Further, the blocker variable subsequence is flanked on its 3' and 5' ends by the target-neutral subsequence and is continuous with the target-neutral subsequence.

Thereafter, a first primer oligonucleotide is introduced to the sample. The first primer oligonucleotide is sufficient to induce enzymatic extension. The first primer oligonucleotide includes a second sequence, which is complementary to a second portion of the homologous subsequence. Further, the second sequence overlaps with the 5' end of the target-neutral subsequence by at least 5 nucleotides such that the second sequence includes an overlapping subsequence and a non-overlapping subsequence. The second sequence does not include any sequence complementary to the variable subsequence.

At the next step, a DNA polymerase, nucleoside triphosphates, and one or more reagents necessary for polymerase-based nucleic acid amplification are introduced to the sample; and the sample is reacted under conditions sufficient to achieve nucleic acid amplification.

An advantage of the present invention is improved mutation sensitivity matching and/or exceeding allele-specific primer approaches.

Another advantage of the present invention is a simple 2-step thermal cycling protocol with significant (8°C) temperature robustness.

Yet another advantage of the present invention is to provide inexpensive DNA primer and blocker reagents without complex backbone or nucleotide modifications.

Yet another advantage of the present invention is compatibility with high fidelity enzymes.

Yet another advantage of the present invention is that the allele-specific blocker binds more strongly to the variant than to the target, so that the non-allele-specific primer more favourably displace blockers bound to target as compared to blockers bound to variant. The advantage of using non-allele-specific primers is that spuriously amplified variants result in amplification products (amplicons) bearing the variant sequence rather than the target sequence. Thus, specificity is compounded at every cycle.

This summary is provided to introduce disclosure, certain aspects, advantages and novel features of the invention in a simplified form that are further described below in the detailed description. This summary is not intended to identify key features or essential features of the claimed subject matter, nor is it intended to be used to limit the scope of the claimed subject matter.

## BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the disclosure is not limited to specific methods and instrumentalities disclosed herein.

FIG. 1 illustrates a schematic representation of a non-allele-specific primer and an allele-specific blocker for allele-specific amplification in accordance with the present invention.

FIG. 2 illustrates a schematic representation of a thermodynamic design of a primer and a blocker in accordance with the present invention.

FIG. 3 illustrates a schematic representation of calculation of $\Delta G°$ values from sequence in accordance with the present invention. FIG. 3 discloses SEQ ID NOS 1, 33, 1, 34, 2, 33, 2, and 34, respectively, in order of appearance.

FIG. 4 illustrates a schematic representation of rare allele detection in human genomic DNA in accordance with the Example 1. FIG. 4 discloses SEQ ID NOS 3-4, 35, 32, 36, and 32, respectively, in order of appearance.

FIG. 5 illustrates a schematic representation of rare temperature robustness in accordance with the Example 2. FIG. 5 discloses SEQ ID NOS 5-6 and 37-38, respectively, in order of appearance.

FIG. 6A illustrates a schematic representation of increased primer design flexibility in accordance with the Example 3. FIG. 6B illustrates a schematic representation of the nucleic acid sequences of 4 sets of primer and blocker pairs that amplifies the human SNP rs3789806 in accordance with the Example 3. FIG. 6B discloses SEQ ID NOS 5-12 and 39-40, respectively, in order of appearance.

FIG. 7A illustrates a schematic representation of simulation of effects of $\Delta G°_{rxn1}$ with fixed $\Delta\Delta G° = 2$ kcal/mol in accordance with the Example 4.

FIG. 7B(I) illustrates a schematic representation of an experimental validation on SMAD7 rs4939827 in accordance with the Example 4. FIG. 7B(I) discloses SEQ ID NOS 3, 13-16, 4, 17, and 35-36, respectively, in order of appearance.

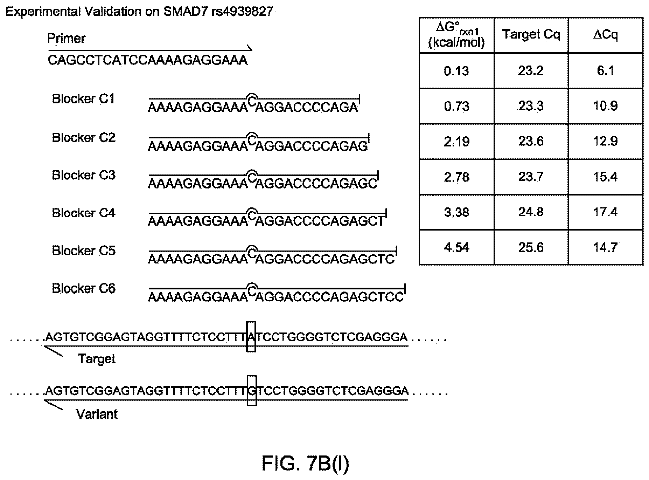

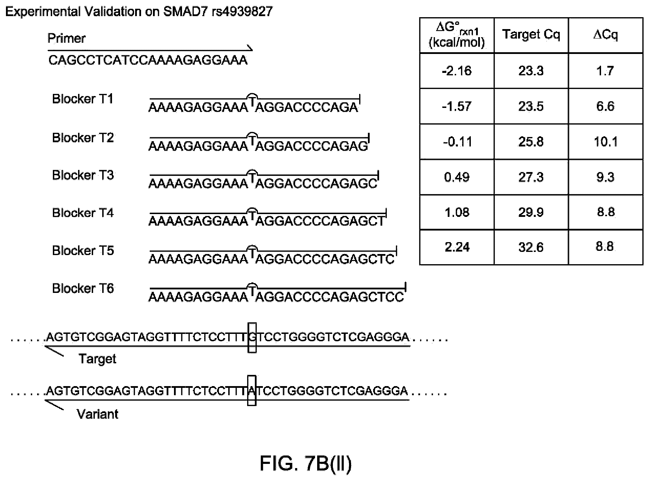

FIG. 7B(II) illustrates a schematic representation of an experimental validation on SMAD7 rs4939827 in accordance with the Example 4. FIG. 7B(II) discloses SEQ ID NOS 3, 18-23, 36, and 35, respectively, in order of appearance.

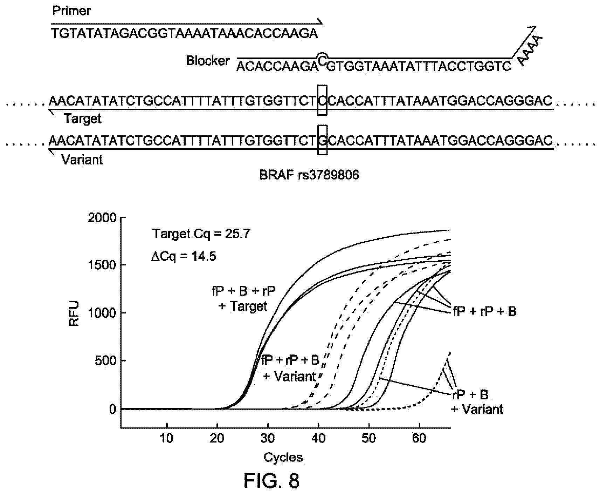

FIG. 8 illustrates a schematic representation of a blocker with 3' non-homologous region in accordance with the Example 5. FIG. 8 discloses SEQ ID NOS 5, 24, and 37-38, respectively, in order of appearance.

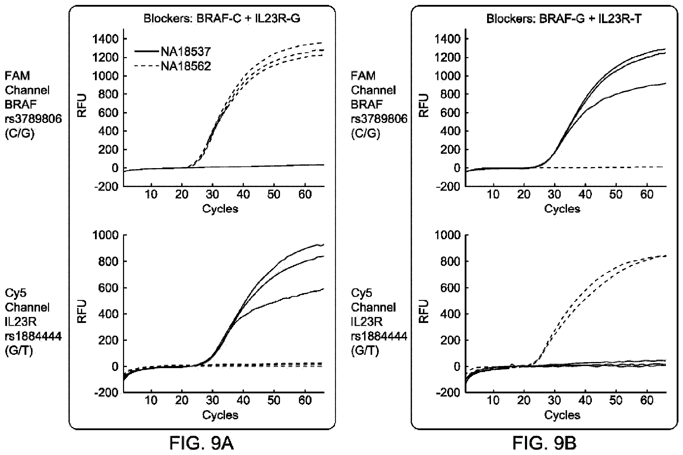

FIG. 9A illustrates a schematic representation of multiplexing using TaqMan® probes in accordance with the Example 6.

FIG. 9B illustrates a schematic representation of multiplexing using TaqMan® probes in accordance with the Example 6.

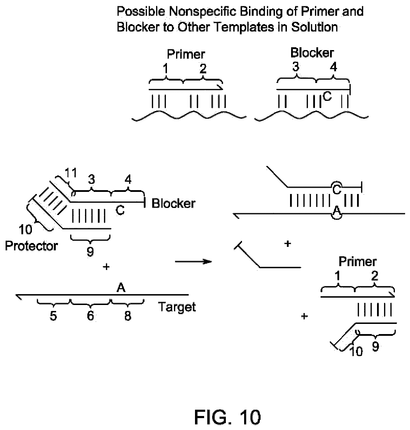

FIG. 10 illustrates a schematic representation of protectors for Primer and Blocker in accordance with the Example 7.

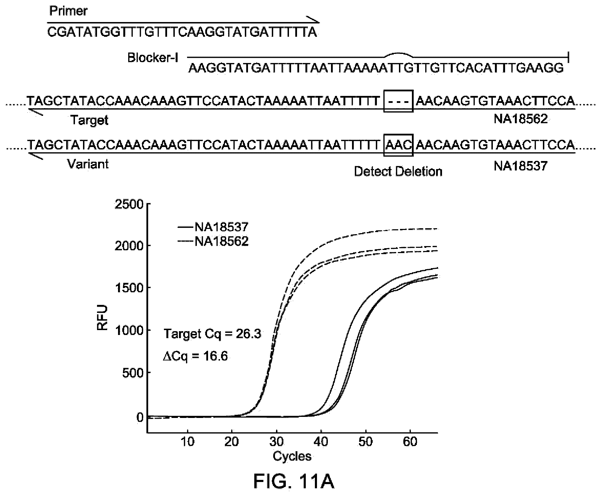

FIG. 11A illustrates a schematic representation of detection of deletion (STAG2 rs200841330) in accordance with the Example 8. FIG. 11A discloses SEQ ID NOS 25-26 and 41-42, respectively, in order of appearance.

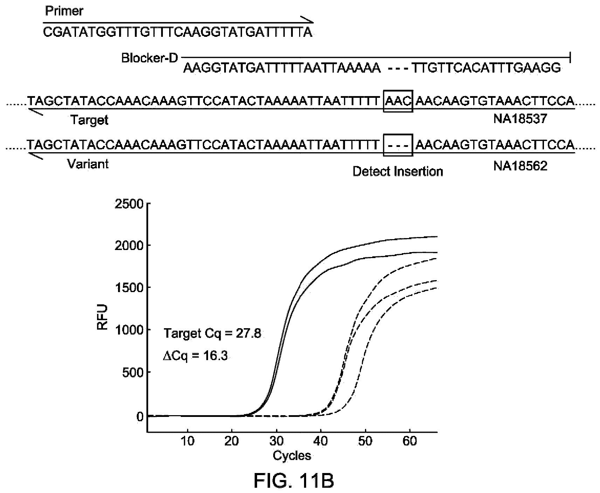

FIG. 11B illustrates a schematic representation of detection of insertion (STAG2 rs200841330) in accordance with the Example 8. FIG. 11B discloses SEQ ID NOS 25, 43, 42, and 41, respectively, in order of appearance.

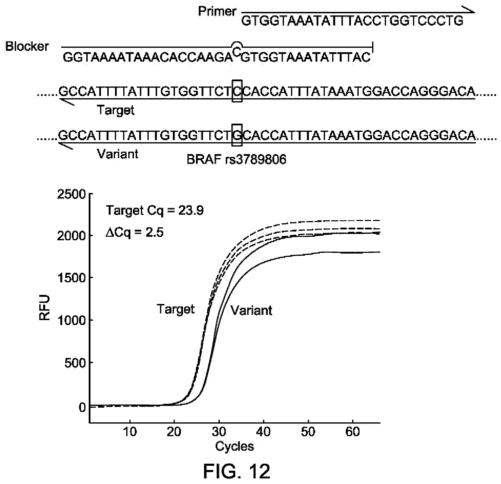

FIG. 12 illustrates a schematic representation of an upstream blocker in accordance with the Example 9. FIG. 12 discloses SEQ ID NOS 27-28 and 44-45, respectively, in order of appearance.

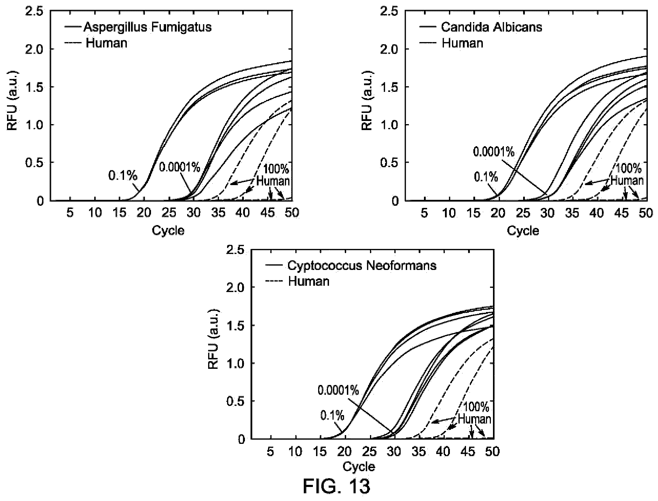

FIG. 13 illustrates a schematic representation of specific amplification of fungal ribosomal RNA-encoding DNA in accordance with the Example 10.

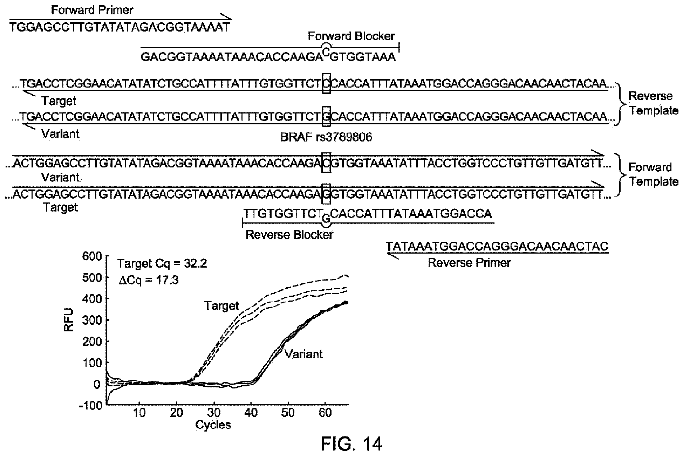

FIG. 14 illustrates a schematic representation of Dual NASP in accordance with the Example 11. FIG. 14 discloses SEQ ID NOS 29, 10, 46-49, and 30-31, respectively, in order of appearance.

## DETAILED **DESCRIPTION** OF THE INVENTION

The present invention will be described with respect to particular embodiments and with reference to certain drawings, but the invention is not limited thereto, but only by claims. The drawings described are only schematic and are non-limiting. In the drawings, the size of some of the elements may be exaggerated or distorted and not drawn on scale for illustrative purposes. Where the elements of the invention are designated as "a" or "an" in first appearance and designated as "the" or "said" for second or subsequent appearances unless something else is specifically stated.

The present invention now will be described more fully here later to with reference to the accompanying drawings, in which some, but not all embodiments of the invention are shown. Indeed, the invention may be embodied in many different forms and should not be construed as limited to the embodiments set forth herein, rather, these embodiments are provided so that this disclosure satisfies all the legal requirements.

*Definition*

Unless otherwise defined, all technical and scientific terms used herein have the same meaning as commonly understood by one of ordinary skill in the art.

As used herein, the term "blocker oligonucleotide" refers to at least one continuous strand of from about 12 to about 100 nucleotides in length and if so indicated herein, may further include a functional group or nucleotide sequence at its 3' end that prevents enzymatic extension during an amplification process such as polymerase chain reaction.

As used herein, the term "primer oligonucleotide" refers to a molecule comprising at least one continuous strand of from about 12 to about 100 nucleotides in length and sufficient to permit enzymatic extension during an amplification process such as polymerase chain reaction.

As used herein, the term "target-neutral subsequence" refers to a sequence of nucleotides that is complementary to a sequence in both a target nucleic acid and a variant nucleic acid. For example, a desired nucleic acid sequence to be targeted for amplification (target nucleic acid) may exist in a sample with a nucleic acid molecule having a predominantly homologous sequence with the target nucleic acid with the exception of a variable region (variant nucleic acid), such variable region in some instance being only a single nucleotide difference from the target nucleic acid. In this example, the target-neutral subsequence is complementary to at least a portion of the homologous sequence shared between the two nucleic acids, but not the variable region. Thus, as used herein, the term "blocker variable subsequence" refers to a nucleotide sequence of a blocker oligonucleotide which is complementary to the variable region of the variant nucleic.

As used herein, the term "overlapping subsequence" refers to a nucleotide sequence of at least 5 nucleotides of a primer oligonucleotide that is homologous with a portion of the blocker oligonucleotide sequence used in a composition as described herein. The overlapping subsequence of the primer oligonucleotide may be homologous to any portion of the target-neutral subsequence of the blocker oligonucleotide, whether 5' or 3' of the blocker variable subsequence. Thus, the term "non-overlapping subsequence" refers to the sequence of a primer oligonucleotide that is not the overlapping subsequence.

As used herein, the term "target sequence" refers to the nucleotide sequence of a nucleic acid that harbours a desired allele, such as a single nucleotide polymorphism, to be amplified, identified, or otherwise isolated. As used herein, the term "variant sequence" refers to the nucleotide sequence of a nucleic acid that does not harbour the desired allele. For example, in some instances, the variant sequence harbours the wild-type allele whereas the target sequence harbours the mutant allele. Thus, in some instance, the variant sequence and the target sequence are derived from a common locus in a genome such that the sequences of each may be substantially homologous except for a region harbouring the desired allele, nucleotide or group or nucleotides that varies between the two.

The present invention relates to an oligonucleotide composition. In an embodiment of the present invention, the oligonucleotide composition includes a non-allele-specific primer oligonucleotide and an allele-specific blocker oligonucleotide for allele-specific amplification.

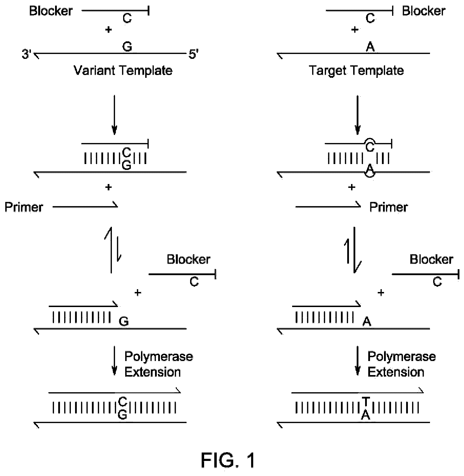

In another embodiment of the present invention, the oligonucleotide composition includes a blocker oligonucleotide and a first primer oligonucleotide. The blocker oligonucleotide includes a first sequence having a target-neutral subsequence and a blocker variable subsequence. The blocker variable subsequence is flanked on its 3' and 5' ends by the target-neutral subsequence and is continuous with the target-neutral subsequence, In other words, the blocker variable subsequence may divide the target-neutral subsequence into two portions, a portion to the 3' of the blocker variable subsequence and a portion to the 5'. The first primer oligonucleotide is sufficient to induce enzymatic extension; herein the first primer oligonucleotide includes a second sequence. The second sequence overlaps with the target-neutral subsequence by at least 5 nucleotides such that the second sequence includes an overlapping subsequence and a non-overlapping subsequence. The second sequence does not include the blocker variable subsequence. Thus, the primer oligonucleotide can be characterized as a non-allele specific primer. The overlapping region can be homologous to a portion of the target-neutral subsequence of the blocker oligonucleotide on either the 5' side of the blocker variable subsequence or on the 3' side of the blocker variable subsequence. As shown in FIG. 1, the primer oligonucleotide overlapping subsequence is homologous with the target-neutral subsequence of the blocker on the 5' side of the blocker variable subsequence (denoted as "C" on the blocker). Here, the target-neutral subsequence is the portions of the blocker on either side of the blocker variable subsequence C. FIG. 12 depicts one example where the overlapping subsequence of the primer is homologous with a portion of target-neutral subsequence of the blocker that is 3' of the blocker variable subsequence C.

In yet another embodiment of the present invention, the blocker oligonucleotide further includes a functional group or a non-complementary sequence region at or near the 3' end, which prevents enzymatic extension. In an instance, the functional group of the blocker oligonucleotide is selected from, but is not limited to, the group consisting of a 3-carbon spacer or a dideoxynucleotide.

The second sequence yields a standard free energy of hybridization ($\Delta G°_{PT}$) and the first sequence yields a standard free energy of hybridization ($\Delta G°_{BT}$), which satisfies the following condition:

$$+2 \text{ kcal/mol} \geq \Delta G°_{PT} - \Delta G°_{BT} \geq -8 \text{ kcal/mol}$$

The non-overlapping subsequence yields a standard free energy of hybridization ($\Delta G°_3$), which satisfies the following condition:

$$-4 \text{ kcal/mol} \geq \Delta G°_3 \geq -12 \text{ kcal/mol}$$

In an instance, the second sequence overlaps with the 5' end of the target-neutral subsequence by about 5 nucleotides to about 40 nucleotides. In another instance, the second sequence overlaps with the 5' end of the target-neutral subsequence by about 7 nucleotides to about 30 nucleotides.

In an instance, the concentration of the blocker oligonucleotide is about 2 to about 10,000 times greater than the concentration of the first primer oligonucleotide. In another instance, the concentration of the blocker oligonucleotide is about 5 to about 1,000 times greater than the concentration of the first primer oligonucleotide.

In yet another embodiment of the present invention, the oligonucleotide composition further includes a second primer oligonucleotide sufficient to induce enzymatic extension. The second primer oligonucleotide includes a third sequence, which does not overlap the first sequence or second sequence, is target-neutral and is sufficient to use with the first primer oligonucleotide to amplify a region of a nucleic acid using polymerase chain reaction.

In yet another embodiment of the present invention, the oligonucleotide composition further includes a second blocker oligonucleotide. The second blocker oligonucleotide includes a fourth sequence having a second target-neutral subsequence and a second blocker variable subsequence. The second blocker variable subsequence has the same sequence as the non-target specific subsequence of the target nucleic acid given that it is typically designed to bind the antisense sequence of the target. The second blocker variable subsequence is flanked on its 3' and 5' ends by the second target-neutral subsequence and is continuous with the second target-neutral subsequence. The third sequence overlaps with the 5' end of the second target-neutral subsequence by at least 5 nucleotides such that the third sequence includes a second overlapping subsequence and a second non-overlapping subsequence.

In yet another embodiment of the present invention, the second blocker oligonucleotide further includes a second functional group or a second non-complementary sequence region at or near the 3' end, which prevents enzymatic extension. In an instance, the second functional group of the second blocker oligonucleotide is selected from, but is not limited to, the group consisting of a 3-carbon spacer or a dideoxynucleotide.

The third sequence yields a standard free energy of hybridization ($\Delta G°_{PT2}$) and the fourth sequence yields a standard free energy of hybridization ($\Delta G°_{BT2}$), which satisfies the following condition:

$$+2 \text{ kcal/mol} \geq \Delta G°_{PT2} - \Delta G°_{BT2} \geq -8 \text{ kcal/mol}$$

The second non-overlapping subsequence yields a standard free energy of hybridization ($\Delta G°_6$), which satisfies the following condition:

$$-4 \text{ kcal/mol} \geq \Delta G°_6 \geq -12 \text{ kcal/mol}.$$

In one instance, the third sequence overlaps with the 5' end of the second target-neutral subsequence by about 5 nucleotides to about 40 nucleotides. In another instance, the third sequence overlaps with the 5' end of the second target-neutral subsequence by about 7 nucleotides to about 30 nucleotides.

In any of the above embodiments, the primer oligonucleotide and blocker oligonucleotide may each be from about 12 nucleotides in length to about 100 nucleotides in length. In any of the above embodiments, the primer oligonucleotide and blocker oligonucleotide may each be from about 15 nucleotides in length to about 90 nucleotides in length. In any of the above embodiments, the primer oligonucleotide and blocker oligonucleotide may each be from about 20 nucleotides in length to about 80 nucleotides in length. In any of the above embodiments, the primer oligonucleotide and blocker oligonucleotide may each be from about 20 nucleotides in length to about 70 nucleotides in length. In any of the above embodiments, the primer oligonucleotide and blocker oligonucleotide may each be from about 20 nucleotides in length to about 60 nucleotides in length. In any of the above embodiments, the primer oligonucleotide and blocker oligonucleotide may each be 21, 22, 23, 24 , 26, 27, 28, 29, 30, 31, 32, 33, 34, 35, 36, 37, 38, 39, 40, 41, 42, 43, 44, 45, 46, 47, 48, 49, or 50 nucleotides in length.

In an instance, the concentration of the second blocker oligonucleotide is about 2 to about 10,000 times greater than the concentration of the second primer oligonucleotide. In another instance, the concentration of the second blocker oligonucleotide is about 5 to about 1,000 times greater than the concentration of the second primer oligonucleotide.. In another instance, the concentration of the second blocker oligonucleotide is about 10 to about 900 times greater than the concentration of the second primer oligonucleotide. In another instance, the concentration of the second blocker oligonucleotide is about 20 to about 800 times greater than the concentration of the second primer oligonucleotide.. In another instance, the concentration of the second blocker oligonucleotide is about 30 to about 700 times greater than the concentration of the second primer oligonucleotide. In another instance, the concentration of the second blocker oligonucleotide is about 40 to about 600 times greater than the concentration of the second primer oligonucleotide. In another instance, the concentration of the second blocker oligonucleotide is about 50 to about 500 times greater than the concentration of the second primer oligonucleotide. In another instance, the concentration of the second blocker oligonucleotide is about 60 to about 400 times greater than the concentration of the second primer oligonucleotide. In another instance, the concentration of the second blocker oligonucleotide is about 70 to about 300 times greater than the concentration of the second primer oligonucleotide. In another instance, the concentration of the second blocker oligonucleotide is about 80 to about 200 times greater than the concentration of the second primer oligonucleotide. In another instance, the concentration of the second blocker oligonucleotide is about 90 to about 100 times greater than the concentration of the second primer oligonucleotide.

In yet another embodiment of the present invention, the oligonucleotide composition further includes a reagent necessary for polymerase chain reaction.

In yet another embodiment of the present invention, the oligonucleotide composition further includes a plurality of nucleoside triphosphates.

In yet another embodiment of the present invention, the oligonucleotide composition further includes a DNA polymerase or an RNA polymerase.

In yet another embodiment of the present invention, the oligonucleotide composition further includes a reagent necessary for polymerase chain reaction, a plurality of nucleoside triphosphates, a DNA polymerase.

In yet another embodiment of the present invention, the blocker variable subsequence is a single nucleotide.

The present invention further relates to a method for amplification of a target sequence. The method includes steps of (a) obtaining a sample containing one or more copies of a first nucleic acid having a variant sequence and at least one copy of a second nucleic acid; herein the second nucleic acid has the target sequence. The target sequence and variant sequence each has a homologous subsequence and a variable subsequence. The variable subsequence further includes at least one nucleotide, but may include 2-5 nucleotides. Furthermore, the variable subsequence of the target sequence is a target-specific subsequence and the variable subsequence of the variant sequence is a non-target specific subsequence.

The method further comprises the step of (b) introducing a blocker oligonucleotide to the sample; herein the blocker oligonucleotide includes a first sequence having a target-neutral subsequence and a blocker variable subsequence. The target-neutral subsequence is complementary to a portion of the homologous subsequence and the blocker variable subsequence is complementary to the non-target specific subsequence. Further, the blocker variable subsequence is flanked on its 3' and 5' ends by the target-neutral subsequence and is continuous with the target-neutral subsequence.

The method further comprises the step of (c) introducing a first primer oligonucleotide to the sample. The first primer oligonucleotide is sufficient to induce enzymatic extension. The first primer oligonucleotide includes a second sequence. Further, the second sequence is complementary to a second portion of the homologous subsequence, and may overlap with the 5' end of the target-neutral subsequence by at least 5 nucleotides such that the second sequence includes an overlapping subsequence and a non-overlapping subsequence. The second sequence does not include any sequence complementary to the variable subsequence.

The method further comprises the step of (d) introducing to the sample a DNA polymerase, nucleoside triphosphates, and one or more reagents necessary for polymerase-based nucleic acid amplification; and then (e) reacting the sample under conditions sufficient to achieve nucleic acid amplification.

In an embodiment of the present invention, the blocker oligonucleotide further includes a functional group or a non-complementary sequence region at or near the 3' end, which prevents enzymatic extension. In an instance, the functional group is selected from, but is not limited to, the group consisting of a 3-carbon spacer or a dideoxynucleotide.

The second sequence yields a standard free energy of hybridization ($\Delta G°_{PT}$) and the first sequence yields a standard free energy of hybridization ($\Delta G°_{BT}$), which satisfies the following condition:

$$+2 \text{ kcal/mol} \geq \Delta G°_{PT} - \Delta G°_{BT} \geq -8 \text{ kcal/mol}$$

The non-overlapping subsequence yields a standard free energy of hybridization ($\Delta G°_3$), which satisfies the following condition:

$$-4 \text{ kcal/mol} \geq \Delta G°_3 \geq -12 \text{ kcal/mol}$$

In one instance, the second sequence overlaps with the 5' end of the target-neutral subsequence of the blocker by about 5 nucleotides to about 40 nucleotides (i.e., the "overlapping subsequence). In other words, the second sequence comprises an overlapping subsequence that is homologous with 5 nucleotides to about 40 nucleotides of the target-neutral subsequence of the blocker that is on the 5' side of the blocker variable subsequence of the blocker. In another instance, the second sequence overlaps with the 5' end of the target-neutral subsequence by about 7 nucleotides to about 30 nucleotides. In yet another instance, the second sequence comprises an overlapping subsequence that is homologous to the portion of the target neutral subsequence of the blocker that is on the 3' side of the blocker variable subsequence of the blocker.

In one instance, the concentration of the blocker oligonucleotide introduced into the sample is about 2 to about 10,000 times greater than the concentration of the first primer oligonucleotide introduced into the sample. In another instance, the concentration of the blocker oligonucleotide introduced into the sample is about 5 to about 1,000 times greater than the concentration of the first primer oligonucleotide introduced into the sample. In another instance, the concentration of the blocker oligonucleotide introduced into the sample is about 10 to about 500 times greater than the concentration of the first primer oligonucleotide introduced into the sample. In another instance, the concentration of the blocker oligonucleotide introduced into the sample is about 20 to about 250 times greater than the concentration of the first primer oligonucleotide introduced into the sample. In another instance, the concentration of the blocker oligonucleotide introduced into the sample is about 40 to about 125 times greater than the concentration of the first primer oligonucleotide introduced into the sample. In another instance, the concentration of the blocker oligonucleotide introduced into the sample is about 50 to about 100 times greater than the concentration of the first primer oligonucleotide introduced into the sample. In another instance, the concentration of the blocker oligonucleotide introduced into the sample is about 300 to about 400 times greater than the concentration of the first primer oligonucleotide introduced into the sample. In another instance, the concentration of the blocker oligonucleotide introduced into the sample is about 500 to about 600 times greater than the concentration of the first primer oligonucleotide introduced into the sample.

In another embodiment of the present invention, the DNA polymerase is a thermostable DNA polymerase.

In yet another embodiment of the present invention, the method further includes the conditions sufficient to achieve nucleic acid amplification by exposing the sample to at least 10 cycles. Each cycle has at least 2 different temperature exposures, one temperature exposure of at least 85°C, and one temperature exposure of no more than 75°C.

In yet another embodiment of the present invention, the method further includes the step of introducing to the sample an enzyme selected from the group consisting of a nicking enzyme, a recombinase, a helicase, a RNAse a reverse transcriptase, or any combination thereof.

In yet another embodiment of the present invention, the method further includes a step of introducing a second primer oligonucleotide into the sample. The second primer oligonucleotide includes a third sequence. Further, the third sequence does not overlap with the variable subsequence. Furthermore, the third sequence is target-neutral and is sufficient to use with the first primer oligonucleotide to amplify a region of a nucleic acid having the target sequence.

In yet another embodiment of the present invention, the method further includes a step of introducing a second blocker oligonucleotide to the sample. The second blocker oligonucleotide includes a fourth sequence; herein the fourth sequence has a second target-neutral subsequence and a second blocker variable subsequence. Further, the second blocker variable subsequence is the complementary sequence to the blocker variable subsequence. Furthermore, the second blocker variable subsequence is flanked on its 3' and 5' ends by the second target-neutral subsequence and is continuous with the second target-neutral subsequence. In addition, the third sequence overlaps with the 5' end of the second target-neutral subsequence by at least 5 nucleotides such that the third sequence includes a second overlapping subsequence and a second non-overlapping subsequence.

In yet another embodiment of the present invention, the second blocker oligonucleotide further includes a second functional group or a second non-complementary sequence region at or near the 3' end, which prevents enzymatic extension. In an instance, the second functional group is selected from, but is not limited to, the group consisting of a 3-carbon spacer or a dideoxynucleotide.

The third sequence yields a standard free energy of hybridization ($\Delta G°_{PT2}$) and the fourth sequence yields a standard free energy of hybridization ($\Delta G°_{BT2}$), which satisfies the following condition:

$$+2 \text{ kcal/mol} \geq \Delta G°_{PT2} - \Delta G°_{BT2} \geq -8 \text{ kcal/mol}$$

The second non-overlapping subsequence yields a standard free energy of hybridization ($\Delta G°_6$), which satisfies the following condition:

$$-4 \text{ kcal/mol} \geq \Delta G°_6 \geq -12 \text{ kcal/mol}$$

In an instance, the third sequence overlaps with the 5' end of the second target-neutral subsequence by about 5 nucleotides to about 40 nucleotides. In another instance, the third sequence overlaps with the 5' end of the second target-neutral subsequence by about 7 nucleotides to about 30 nucleotides.

In an instance, the concentration of the second blocker oligonucleotide introduced into the sample is about 2 to about 10,000 times greater than the concentration of the second primer oligonucleotide introduced into the sample. In another instance, the concentration of the second blocker oligonucleotide introduced into the sample is about 5 to about 1,000 times greater than the concentration of the second primer oligonucleotide introduced into the sample.

In yet another embodiment of the present invention, the method for amplification of a target sequence further includes a step of removing an aliquot from the sample; and repeating steps (b) through (e) defined above.

FIG. 1 illustrates a schematic representation of the non-allele-specific primer and the allele-specific blocker for allele-specific amplification. A nucleic acid reagent mixture comprises two oligonucleotide species, a primer (primer oligonucleotide) and a variant specific blocker (blocker oligonucleotide), which act together to enable reliable rare target amplification in conjunction with a polymerase-based amplification method. The blocker may be modified at the 3' end with a non-extensible modification, such as a dideoxynucleotide or a 3-carbon linker. The sequences of the primer and blocker are rationally designed based on the thermodynamics of their hybridization to the target nucleic acid sequence and the variant nucleic acid sequence. In some embodiments, the blocker is present in a significantly higher concentration than the primer, so that the preponderance of the target and the variant nucleic acid sequences bind to blocker before binding to primer. The primer binds transiently to the blocker-target or blocker-variant molecules, and possesses a probability for displacing the blocker in binding to the target or variant. Because the blocker sequence is specific to the variant target, its displacement from the variant is less thermodynamically favourable than its displacement from the target. Thus, the non-allele-specific primer amplifies the target sequence with higher yield/efficiency than it amplifies the variant sequence. The non-allele-specific nature of the primer means that spuriously amplified variant sequence bear the variant allele, rather than the target allele, so that subsequent amplification cycles also exhibit amplification bias in favour of the target.

High concentration of the variant-specific blocker results in higher probability of blocker binding to both the target and the variant first, before the primer has an opportunity to bind. The primer initiates binding, to both the target-blocker or variant-blocker complex, via a unique region not overlapping with the blocker, and subsequently possibly displaces the blocker via a process of enzyme-free strand displacement.

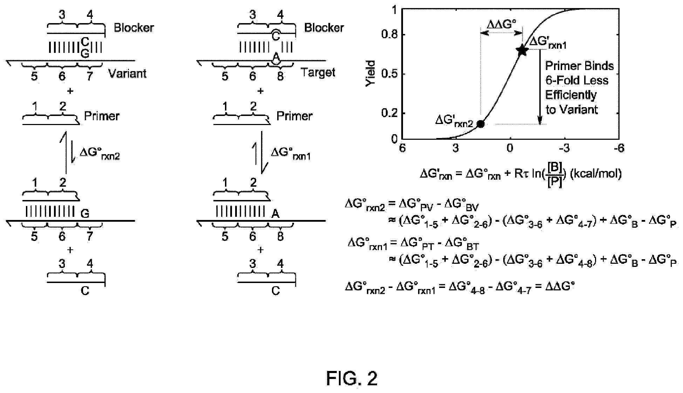

FIG. 2 illustrates a schematic representation of a thermodynamic design of a primer and a blocker. The sequences of the primer and the blocker are rationally designed to achieve desirable reaction thermodynamics. Here, the primer, the blocker, the target, and the variant nucleic acid sequences are subdivided into regions comprising continuous nucleotides, denoted by the numbers 1 through 8 in Fig. 2. The standard free energy of hybridization between the primer and the target ($\Delta G^\circ_{PT}$) and the standard free energy of hybridization between the blocker and the target ($\Delta G^\circ_{BT}$) satisfies:

$$+2 \text{ kcal/mol} \geq \Delta G^\circ_{PT} - \Delta G^\circ_{BT} \geq -8 \text{ kcal/mol}$$

The primer and blocker systems that satisfy the above inequality generate a large difference in the hybridization yields between primer bound to the target and primer bound to the variant in each cycle, resulting in a target-specific amplification.

The process of enzyme-free strand displacement is guided by the relative thermodynamics of the primer binding versus the blocker binding. The blocker hybridizes more favourably to the variant (standard free energy of binding $\Delta G^\circ_{BV}$) than to the target (standard free energy of binding $\Delta G^\circ_{BT}$, $\Delta G^\circ_{BT} > \Delta G^\circ_{BV}$ because more negative $\Delta G^\circ$ indicates stronger favourability). In another embodiment of the present invention, the primer hybridizes equally favourably to the variant (standard free energy of binding $\Delta G^\circ_{PV}$) as to the target (standard free energy of binding $\Delta G^\circ_{PT}$, $\Delta G^\circ_{PV} = \Delta G^\circ_{PT}$).

The reaction of the primer displacing the blocker in binding to the target, BT + P $\Leftrightarrow$ PT + B, has a standard free energy $\Delta G^\circ_{ixn1} = \Delta G^\circ_{PT} - \Delta G^\circ_{BT}$, which is more negative (more favourable) than that of the reaction of the primer displacing the blocker in binding to the variant, BV + P $\Leftrightarrow$ PV + B, which has standard free energy $\Delta G^\circ_{rxn2} = \Delta G^\circ_{PV} - \Delta G^\circ_{BV}$. For good performance of the present invention, the primer and blocker usually should be designed so that $\Delta G^\circ_{rxn1} \leq 0$ and $\Delta G^\circ_{rxn2} \geq 0$. However, because the relative concentrations of the blocker and the primer can influence the equilibrium distribution and effective thermodynamics of the reaction, the $\Delta G^\circ_{rxn1}$ and $\Delta G^\circ_{rxn2}$ guidelines are not absolute.

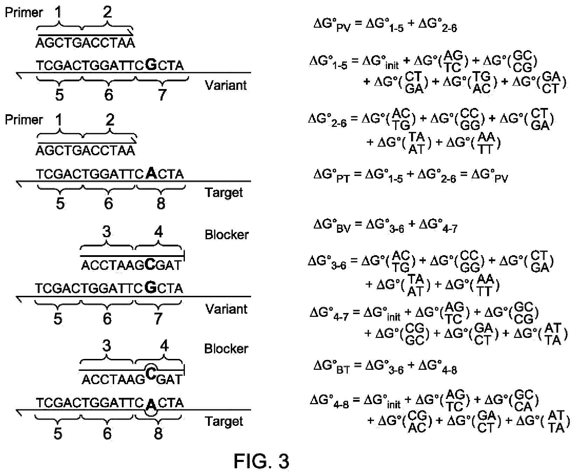

FIG. 3 illustrates a schematic representation of calculation of $\Delta G^\circ$ values from sequence. There exist different conventions for calculating the $\Delta G^\circ$ of different region interactions; shown in FIG. 3 are exemplary energy calculations based on the nearest neighbour model. The $\Delta G^\circ_{1-5}$, $\Delta G^\circ_{4-7}$, and $\Delta G^\circ_{4-8}$ terms further include the standard free energy of hybridization initiation ($\Delta G^\circ_{init}$), and the extra base stack adjoining the sixth region. Standard free energies, $\Delta G^\circ$, calculated using other methods may result in the same $\Delta G^\circ_{PT}$, $\Delta G^\circ_{PV}$, $\Delta G^\circ_{BT}$, and $\Delta G^\circ_{\beta V}$ values, though the thermo-dynamics of individual regions (e.g. $\Delta G^\circ_{3-6}$) may differ. The illustrative examples are intended to show the method of $\Delta G^\circ$ calculation used in the present invention, and are not intended to be suggestive of sequences for an assay; the listed sequences may be too short to stably bind. The calculation of $\Delta G^\circ_{PT}$, $\Delta G^\circ_{PV}$, $\Delta G^\circ_{BT}$, and $\Delta G^\circ_{BV}$ from the primer sequence, blocker sequence, target sequence, variant sequence, operational temperature, and operational buffer conditions are known to those skilled in the art.

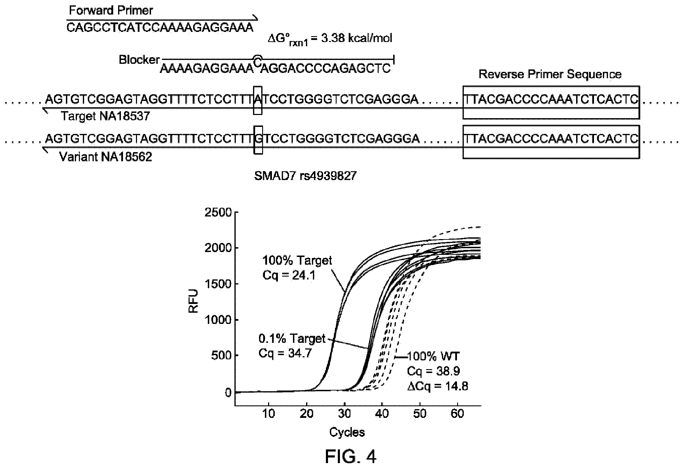

FIG. 4 shows a probe and a blocker design of the present invention and the subsequences around SMAD7 gene locus of two human repository samples NA18537 and NA18562. The design of a primer blocker set designed to specifically amplify the A variant of the SMAD7 single nucleotide polymorphism (SNP). The blocker is designed to be perfectly complementary to the variant template bearing a G-variant of the SMAD7 SNP, and is functionalized at the 3' end with a 3-carbon linker (C3) that prevents enzymatic extension by the Taq DNA polymerase. A reverse primer binds to the 3' of the forward primer, and is not specific to either allele. For the sake of explanation of terminology used to describe various subsequences of the primar and blocker, the oligonucleotides can be described as follows. The blocker oligonucleotide comprises a target-neutral subsequence which includes the sequence to the 5' and 3' of the cytosine nucleotide that is depicted out of alignement with the remainder of the sequence - the cytosine nucleotide in this instance represents the blocker variable subsequence of the blocker. The overlapping subsequence of the primer oligonucleotide comprises sequence that is homologous to the blocker sequence on the 5' side of the blocker variable subsequence (the cytosine).

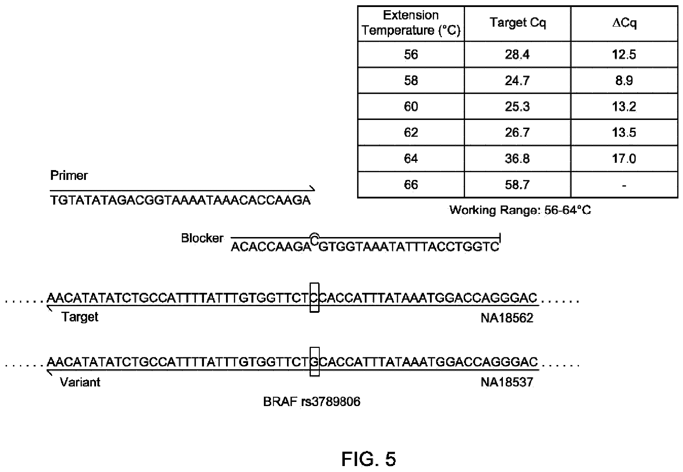

FIG. 5 shows the performance of a primer-blocker pair targeting human SNP rs3789806 (C/G) C allele at annealing/extension temperature ranging from 56°C to 66°C.

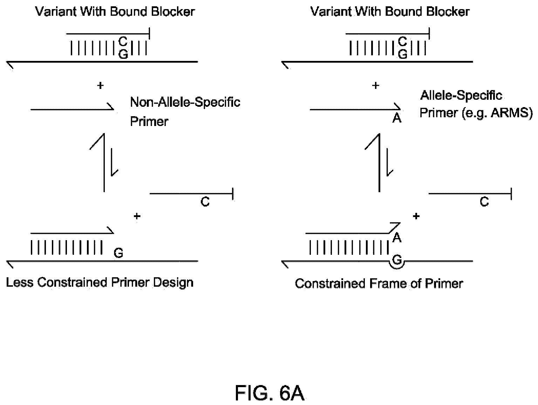

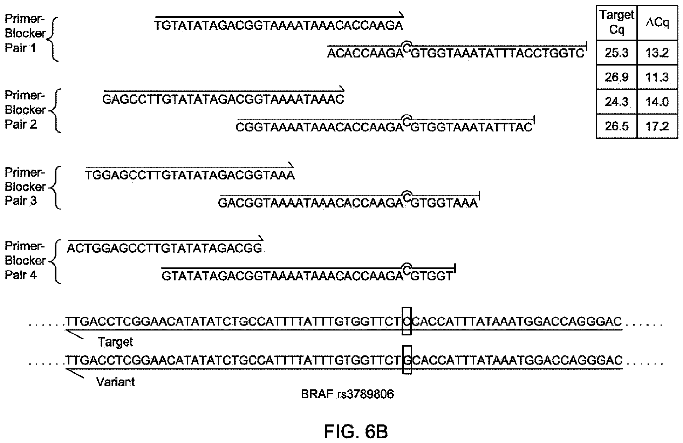

FIG. 6A and FIG. 6B shows primer designs with more design space to avoid undesired interactions.

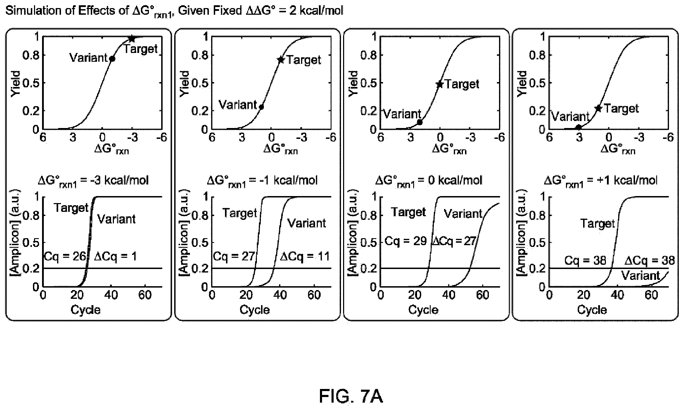

FIG. 7A, and FIG. 7B shows controlling the allele-specific PCR behaviour via design of $\Delta G^\circ_{rxn1}$. Simulations in FIG. 7A suggest that when the primer and blocker are designed so that $\Delta G^\circ_{rxn1}$ is more positive, $\Delta Cq$ values are greater. However, at more positive values of $\Delta G^\circ_{rxn1}$, the amplification of the target is also slowed, resulting in a larger value of Cq. Simulations assume a fixed $\Delta \Delta G^\circ$ of 2 kcal/mol. Top sub graphs show the predicted effects of the different values of $\Delta G^\circ_{rxn1}$ (stars) and $\Delta G^\circ_{rxn2}$ (dots) on the hybridization yields. The bottom subgraphs show simulated amplification curve of the target and the variant for each primer and blocker pair. In all cases depicted, the concentration ratio of blocker to primer is 20:1. FIG. 7B(I) and FIG. 7B(II) show experimental results of $\Delta G^\circ_{rxn1}$ effects on amplification selectivity. Different $\Delta G^\circ_{rxn1}$

values were achieved via lengthening or shortening the 3' end of the blocker. As expected, more positive $\Delta G^{\circ}_{rxn1}$ results in larger Cq for both the target and the variant. We observed an optimal intermediate $\Delta G^{\circ}_{rxn1}$ resulting in largest $\Delta Cq$ and relatively small Cq delay (Cq < 28) for the target. Unlike the simulations, $\Delta Cq$ did not increase monotonically with $\Delta G^{\circ}_{rxn1}$. The formation of primer-dimers and nonspecific amplification produces a Cq ceiling.

The kinetic simulations of the PCR process expressed as:

$$\text{Tfn+1} = \text{Tfn} + \text{Pn} \cdot [\text{Pn} / (\text{Pn} + \text{Bn}) + Y(\Delta G^{\circ}_{rxn1}) \cdot \text{Bn} / (\text{Pn} + \text{Bn})] \cdot \text{Trn}$$

$$\text{Trn+1} = \text{Trn} + \text{Rn} \cdot \text{Tfn}$$

$$\text{Vfn+1} = \text{Vfn} + \text{Pn} \cdot [\text{Pn} / (\text{Pn} + \text{Bn}) + Y(\Delta G^{\circ}_{rxn2}) \cdot \text{Bn} / (\text{Pn} + \text{Bn})] \cdot \text{Vrn}$$

$$\text{Vrn+1} = \text{Vrn} + \text{Rn} \cdot \text{Vfn}$$

where Tfn is the normalized concentration of the forward strand of the target at cycle n, Trn is the normalized concentration of the reverse strand of the target at cycle n, Vfn is the normalized concentration of the forward strand of the variant at cycle n, Vrn is the normalized concentration of the forward strand of the variant at cycle n, Pn is the normalized concentration of the forward primer at cycle n, Bn is the normalized concentration of the blocker at cycle n, Rn is the normalized concentration of the reverse primer at cycle n, and $Y(\Delta G^{\circ})$ is the hybridization yield of a displacement reaction given $\Delta G^{\circ}$ reaction standard free energy. Simulation results are shown in FIG. 7A.

FIG. 8 shows the blocker with a non-homologous sequence at the 3' end rather than a non-extensible chemical functionalization. Because the non-homologous sequences do not bind to the downstream region of either the target or the variant, the non-homologous region effectively prevents enzymatic extension. The target Cq and $\Delta Cq$ values are similar to those shown in Example 1, indicating that like functional groups such as a 3-carbon spacer or a didexoynucleotide, a non-homologous sequence at the 3' end can effectively prevent enzymatic extension Here fP, rP, and B represent the forward primer, the reverse primer, and the blocker (for the forward primer), respectively.

In standard blocking PCR, only the forward primer is biased to amplify the target sequence; the reverse primer amplifies both the target and the variant roughly equally. Amplification specificity can be further improved (with roughly a quadratic improvement in mutation sensitivity) if both the forward and the reverse primers were biased to amplify only the target sequence. To achieve dual target-specific amplification, the forward and reverse primers are designed to be separated by a short distance, so that they overlap with a variant-specific blocker. In the limit, the primer-binding regions are separated by a single nucleotide whose identity varies between the target and the variant. The forward and reverse variant-specific blockers are partially complementary to each other because they bind to complementary strands of the target, and both span the variation region. The blockers are designed so that, despite their partial complementarity, at the operational conditions, the majority of blocker molecules are not hybridized to each other.

FIG. 9A and FIG. 9B, each shows a primer-blocker systems designed for human BRAF rs3789806 and IL23R rs1884444 SNPs were multiplexed in the same reaction. TaqMan® probes targeting the corresponding downstream of the blocker binding regions were designed and used as the readout mechanism.

FIG. 10 shows nonspecific binding of primer or blocker to other primers, blockers, or templates results in nonspecific amplification. The lower panel provides a design of a protector that suppresses the nonspecific binding of the primer and blocker. Protector oligonucleotides are partially complementary to the primer and blocker sequences. The presence of a stoichiometric excess of protectors result in both the primer and the blocker being partially double-stranded. The blocker possesses a new region 11, whose sequence is non complementary to region 5 on the nucleic acid sequence, and the protector possesses a new region 10, whose sequence is complementary to region 11.

FIG. 11A and FIG. 11B, each shows primer and blocker designs for detecting a deletion and an insertion. The primer is non-target-specific, this method can also be used to detect deletion or insertion with uncertain number of bases or uncertain position.

FIG. 12 shows an inferior implementation of the present invention by placing the primer at the 3' of the SNP position and blocker at the 5' of the primer. This implementation generates hybridization specificity only in the first cycle, so the $\Delta Cq$ is smaller than the preferred design.

FIG. 13 demonstrates selective amplification of fungal 18S DNA subsequences of 3 pathogenic fungi species in large excess of homologous human DNA. Because fungal 18S subsequences and its homologous human subsequence differ in multiple regions, we designed corresponding blocker species for both forward primer and reverse primer to maximize amplification specificity.

FIG. 14 describes the use of target-specific amplification for both the forward and the reverse primer, by using two sets of variant-allele blockers, one for each primer. The blockers are partially complementary to each other, but at the operational conditions are not predominately hybridized to one another. The blockers and primers are designed so that the forward primer cannot extend off the reverse blocker, and the reverse primer cannot extend off the forward blocker. FIG. 14 shows preliminary experimental results.

The primer and blocker combinations may be applied to other enzymatic amplification assays for nucleic acids, including but not limited to Nicking Enzyme Amplification Reaction (NEAR), Loop-mediated Isothermal Amplification (LAMP), and Rolling Circle Amplification (RCA).

The present invention is particularly suitable for the detection of a small amount of target in a large excess of variant. For cancer diagnostics applications of the present invention, the variant may refer to the wildtype DNA sequence, and the target may refer a cancer DNA sequence. For infectious disease diagnostics applications of the present invention, the variant may refer to the pathogen DNA sequence, and the target may refer to a homologous human DNA sequence.

*Examples*

Example 1

FIG. 4 shows an Example 1 of the present invention for the probe and the blocker design and the subsequences around SMAD7 gene locus of two human repository samples NA18537 and NA18562. Healthy human single nucleotide polymorphisms (SNP) rs4939827 (CIT) in SMAD7 gene locus were chosen for proof-of-concept validations. T allele as target and C allele variant were arbitrarily designated. Human repository genomic DNA samples NA18562 (homozygous of C allele) and NA18537 (homozygous of T allele) were purchased from Coriell®, and the corresponding nucleotide sequences were obtained from 1000 Genomes website. To prevent unintended polymerization, the blocker was modified with a C3 spacer at the 3' end. qPCR experimental results distinguished 0.1% target (99.9% variant) from 0% target (100% variant). The 0.1% target sample was prepared by mixing 0.1% NA18537 with 99.9% NA18562. The average difference of quantification cycle ($\Delta$Cq) between 100% variant and 100% target was 14.8, and that between 100% variant (denoted as "WT" in the graph) and 0.1% target was 4.2. All experiments were performed in a 96-well plate in the Bio-Rad CFX96™ qPCR machine. In each well, 200 nM primer, 2 $\mu$M blocker, and 20 ng human genomic DNA were mixed in the Bio-rad iTaq™ SYBR® Green Supermix. After a 3 min initiation process at 95°C, 65 cycles of denaturing step at 95°C for 10 seconds and annealing/extension step at 60°C for 30 seconds were performed. Example experiments shown in FIG. 5-12 used a similar protocol.

Table-1

| Gene | SNP | Blocker | Cq numbers | | |
|---|---|---|---|---|---|
| | | | NA18537 | NA18562 | $\Delta$Cq |
| BRAF | rs3789806 G or C | - | 23.2 | 23.4 | 0.2 |
| | | C | 38.5 | 25.3 | 13.2 |
| | | G | 30.1 | 42.7 | 12.6 |
| EGF | rs11568849 C or A | - | 22.5 | 22.3 | 0.2 |
| | | A | 34.7 | 26.3 | 8.4 |
| | | C | 24.7 | 39.33 | 14.6 |
| IL23R | rs1884444 G or T | - | 22.2 | 22.4 | 0.2 |
| | | T | 36.1 | 24 | 12.1 |
| | | G | 28.3 | 35.4 | 7.1 |
| ALK | rs2246745 A or T | - | 22.6 | 22.3 | 0.3 |
| | | A | 23.2 | 33 | 9.8 |
| | | T | 33.2 | 24.1 | 9.1 |

Table-1 shows examples of allele specific amplification results for four set of SNP pairs in human genomic DNA samples NA18562 and NA18537, drawn from the 1000 Genomes database. For each SNP pair, a primer and blocker design for each allele variant were designed and tested. The base identities shown in the "Blocker" column indicates the allele that was being suppressed (variant), and "-" means no blocker was added. In all cases, large $\Delta$Cq values between the two alleles, ranging from 7.1 to 14.6 were observed. All the experiments were performed in triplicates using 400 nM of each

primer and 4 $\mu$M of blocker in Bio-rad iTaq™ SYBR® Green Supermix. The performance of thermodynamically driven designs without any empirical optimization of primer and blocker sequences or experimental conditions were shown by the results.

Example 2

FIG. 5 shows an Example 2 of the present invention for the performance of a primer-blocker pair targeting human SNP rs3789806 (C/G) C allele at annealing/extension temperature ranging from 56°C to 66°C. The Example 2 showed good allele specific amplification from 56°C to 64°C and indicated at least 8°C of temperature robustness. 400 nM of each primer and 4 $\mu$M blocker were used, and other experimental conditions other than annealing/extension temperature were the same as described in Example 1.

Example 3

FIG. 6A and FIG. 6B show an Example 3 of the present invention. The designs of the Primers shown in the present invention provided more design space to avoid undesired interactions. The nucleic acid sequences of four sets of primer and blocker pairs that each specifically amplifies the human SNP rs3789806 C allele exhibited $\Delta C\eta > 11$. 400 nM of each primer and 4 $\mu$M blocker were used, and other experimental conditions were the same as described in Example 1.

Example 4

FIG. 7A, FIG. 7B(I) and FIG. 7B(II) show an Example 4 of the present invention for controlling the allele-specific PCR behaviour via design of $\Delta G°_{rxn1}$. Simulations in FIG. 7A suggested that when the primer and blocker were designed so that $\Delta G°_{rxn1}$ was more positive, $\Delta Cq$ values were greater. However, at more positive values of $\Delta G°_{rxn1}$, the amplification of the target was slowed and resulted in a larger value of Cq. Simulations were assumed as fixed $\Delta\Delta G°$ of 2 kcal/mol. The top sub graphs predicted the effects of the different values of $\Delta G°_{rxn1}$ (stars) and $\Delta G°_{rxn2}$ (dots) on the hybridization yields. The bottom sub graphs predicted simulated amplification curve of the target and the variant for each primer and blocker pair. In all cases depicted, the concentration ratio of blocker to primer was 20:1. FIG. 7B(I) and FIG. 7B(II) show experimental results of $\Delta G°_{rxn1}$ effects on amplification selectivity. Different $\Delta G°_{rxn1}$ values were achieved via lengthening or shortening the 3' end of the blocker. As expected, more positive $\Delta G°_{rxn1}$ resulted in larger Cq for both the target and the variant. An optimal intermediate $\Delta G°_{rxn1}$ resulted in largest $\Delta Cq$ and relatively small Cq delay (Cq < 28) for the target. Unlike the simulations, $\Delta Cq$ did not increase monotonically with $\Delta G°_{rxn1}$ because of the formation of primer-dimers and nonspecific amplification produced by a Cq ceiling. 200 nM of each primer and 2 $\mu$M blocker were used, and other experimental conditions were same as described in Example 1.

Example 5

FIG. 8 shows an Example 5 of the present invention for the blocker with a non-homologous sequence at the 3' end rather than a non-extensible chemical functionalization. Because the non-homologous sequences do not bind to the downstream region of either the target or the variant, the non-homologous region effectively prevents enzymatic extension. 400 nM of each primer and 4 $\mu$M blocker were used, and other experimental conditions were the same as described in Example 1. The target Cq and $\Delta Cq$ values were similar to those shown in FIG. 4, indicating that like functional groups such as a 3-carbon spacer or a didexoynucleotide, a non-homologous sequence at the 3' end effectively prevents enzymatic extension. In FIG. 8, fP, rP, and B represent the forward primer, the reverse primer, and the blocker (for the forward primer), respectively.

Example 6

FIG. 9A and FIG. 9B show an Example 6 of the present invention. For a real-time PCR implementation of our non-allele-specific primer and allele-specific blocker, a reverse primer was also needed, which was not allele-specific. SYBR® Green Supermix and TaqMan® were used in the experiments and the results were not significantly different between the two. The Primer-blocker systems designed for human BRAF rs3789806 and IL23R rs1884444 SNPs were multiplexed in the same reaction. TaqMan® probes targeting the corresponding downstream of the blocker binding regions were designed and used as the readout mechanism. The TaqMan® probe for BRAF rs3789806 amplicons was modified with a FAM fluorophore, an Iowa Black FQ quencher, and an internal ZEN quencher, and the TaqMan® probe for IL23R rsl 884444 amplicons was modified with a Cy5 fluorophore and an Iowa Black RQ quencher. FIG. 9A shows the amplification results of multiplexed detection of BRAF rs3789806 G allele and IL23R rs1884444 T allele. The base identities denoted which allele the blockers were suppressing (variant). FIG. 9B shows multiplexed detection of BRAF rs3789806 C allele and IL23R rs1884444 G allele. In each experiment, the genomic DNA sample was mixed with 400 nM of each primer, 4 μM of each blocker, and 200 nM of each TaqMan® probe in Bio-rad iQ Supermix. The qPCR protocol was the same as indicated in Example 1.

Example 7

FIG. 10 shows an Example 7 of the present invention for nonspecific binding of primer or blocker to other primers, blockers, or templates. The Example 7 resulted in nonspecific amplification. The lower panel provided a design of a protector that suppressed the nonspecific binding of the primer and blocker. The blocker possessed a new region 11, whose sequence was non complementary to region 5 on the nucleic acid sequence, and the protector possessed a new region 10, whose sequence was complementary to region 11.

Example 8

FIG. 11A and FIG. 11B show an Example 8 of the present invention for primer and blocker designs for detecting a deletion and an insertion. Del rs200841330 was used to show proof-of-concept results. 400 nM of each primer and 4 μM blocker were used, and other experimental conditions were same as indicated in Example 1. Since the primer is non-allele-specific, this method can also be used to detect deletion or insertion with uncertain number of bases or uncertain position.

Example 9

FIG. 12 shows an Example 9 of the present invention by placing the primer to the 3' of the SNP position and blocker to the 5' of the primer. The Example 9 generated hybridization specificity only in the first cycle, so the ΔCq was smaller than the preferred design. The primer concentration was 400 nM, and the blocker concentration was 4 μM. Experimental conditions were the same as indicated in Example 1.

Example 10

FIG. 13 shows an Example 10 of the present invention for selective amplification of fungal 18S DNA subsequences of 3 pathogenic fungi species in large excess of homologous human DNA. Because fungal 18S subsequences and its homologous human subsequence differ in multiple regions, corresponding blocker species for both forward primer and reverse primer to maximize amplification specificity were designed. gBlock fragments (sequence verified 400bp double-stranded DNA, IDT) of 18S subsequences from 3 fungi species and the consensus sequence in human were used in the experiments. 60,000 copies (0.1%), 60 copies (0.0001%) and 0 copies (100% Human) of each fungal DNA gBlock fragment were separately mixed with 60,000,000 copies of human DNA. In all cases, results detected rare sequences down to 1 part in 1 million. 200 nM of each primer and 1 μM of each blocker were used, and other experimental conditions were the same as indicated in Example 1.

Example 11

FIG. 14 shows an Example 11 of the present invention. FIG. 14 describes the use of allele-specific amplification for both the forward and the reverse primer; two sets of variant-allele blockers, one for each primer were used. The blockers were partially complementary to each other, but at the operational conditions did not support predominant hybridization to one another. The blockers and primers were designed so that the forward primer cannot extend off the reverse blocker, and the reverse primer cannot extend off the forward blocker. FIG. 14 displayed preliminary experimental results.

The foregoing description of specific embodiments of the present disclosure has been presented for purpose of illustration and description. The exemplary embodiment was chosen and described in order to best explain the principles of the invention and its practical application, to thereby enable others skilled in the art to best utilize the invention and various embodiments with various modifications are suited to the particular use contemplated.

Embodiments of the invention will now be described in the following numbered paragraphs:

1. An oligonucleotide composition comprising:

a blocker oligonucleotide comprising a functional group or a non-complementary sequence region at or near the 3' end, which prevents enzymatic extension, wherein the blocker oligonucleotide comprises a first sequence comprising a target-neutral subsequence and a blocker variable subsequence, wherein the blocker variable subsequence is flanked on its 3' and 5' ends by the target-neutral subsequence and is continuous with the target-neutral subsequence; and

a first primer oligonucleotide sufficient to induce enzymatic extension, wherein the first primer oligonucleotide comprises a second sequence, wherein the second sequence overlaps the target-neutral subsequence by at least 5 nucleotides such that the second sequence comprises an overlapping subsequence and a non-overlapping subsequence, and wherein the second sequence does not include the blocker variable subsequence.

2. The oligonucleotide composition of paragraph 1 wherein the functional group comprises a 3-carbon spacer or a dideoxynucleotide.

3. The oligonucleotide composition of paragraph 1 wherein the overlapping subsequence comprises a portion of the 5' end of the target-neutral subsequence, wherein said portion is from about 5 nucleotides to about 40 nucleotides.

4. The oligonucleotide composition of paragraph 1 wherein the overlapping subsequence comprises a portion of the 5' end of the target-neutral subsequence, wherein said portion is from about 7 nucleotides to about 30 nucleotides.

5. The oligonucleotide composition of paragraph 1 wherein the second sequence yields a standard free energy of hybridization ($\Delta G^\circ_{PT}$) and the first sequence yields a standard free energy of hybridization ($\Delta G^\circ_{BT}$), which satisfies the following condition:

$$+2 \text{ kcal/mol} \geq \Delta G^\circ_{PT} - \Delta G^\circ_{BT} \geq -8 \text{ kcal/mol}.$$

6. The oligonucleotide composition of paragraph 1 wherein the non-overlapping subsequence yields a standard free energy of hybridization ($\Delta G^\circ_3$), which satisfies the following condition:

$$-4 \text{ kcal/mol} \geq \Delta G^\circ_3 \geq -12 \text{ kcal/mol}.$$

7. The oligonucleotide composition of paragraph 1 wherein the concentration of the blocker oligonucleotide is about 2 to about 10,000 times greater than the concentration of the first primer oligonucleotide.

8. The oligonucleotide composition of paragraph 1 wherein the concentration of the blocker oligonucleotide is about 5 to about 1,000 times greater than the concentration of the first primer oligonucleotide.

9. The oligonucleotide composition of paragraph 1 further comprising a second primer oligonucleotide sufficient to induce enzymatic extension, wherein the second primer oligonucleotide comprises a third sequence, wherein the third sequence does not overlap the first sequence or second sequence, and wherein the third sequence is target-neutral and is sufficient to use with the first primer oligonucleotide to amplify a region of a nucleic acid using polymerase chain reaction.

10. The oligonucleotide composition of paragraph 9 further comprising a second blocker oligonucleotide comprising a second functional group or a second non-complementary sequence region at or near the 3' end, which prevents enzymatic extension, wherein the second blocker oligonucleotide comprises a fourth sequence comprising a second target-neutral subsequence and a second blocker variable subsequence, wherein the second blocker variable subsequence is the complementary sequence to the blocker variable subsequence, wherein the second blocker variable subsequence is flanked on its 3' and 5' ends by the second target-neutral subsequence and is continuous with the second target-neutral subsequence, wherein the third sequence overlaps the second target-neutral subsequence by at least 5 nucleotides such that the third sequence comprises a second overlapping subsequence and a second non-overlapping subsequence, and wherein the third sequence does not include the second blocker variable subsequence.

11. The oligonucleotide composition of paragraph 10 wherein the second functional group comprises a 3-carbon spacer or a dideoxynucleotide.

12. The oligonucleotide composition of paragraph 10 wherein the second overlapping subsequence comprises a portion of the 5' end of the second target-neutral subsequence, wherein said portion is from about 5 nucleotides to about 40 nucleotides.

13. The oligonucleotide composition of paragraph 10 wherein the second overlapping subsequence comprises a portion of the 5' end of the second target-neutral subsequence, wherein said portion is from about 7 nucleotides to about 30 nucleotides.

14. The oligonucleotide composition of paragraph 10 wherein the third sequence yields a standard free energy of hybridization ($\Delta G^\circ_{PT2}$) and the fourth sequence yields a standard free energy of hybridization ($\Delta G^\circ_{BT2}$), which satisfies the following condition:

$$+2 \text{ kcal/mol} \geq \Delta G^\circ_{PT2} - \Delta G^\circ_{BT2} \geq -8 \text{ kcal/mol}.$$

15. The oligonucleotide composition of paragraph 10 wherein the second non-overlapping subsequence yields a standard free energy of hybridization ($\Delta G^\circ_6$), which satisfies the following condition:

$$-4 \text{ kcal/mol} \geq \Delta G°_3 \geq -12 \text{ kcal/mol}.$$

16. The oligonucleotide composition of paragraph 10 wherein the concentration of the second blocker oligonucleotide is about 2 to about 10,000 times greater than the concentration of the second primer oligonucleotide.

17. The oligonucleotide composition of paragraph 10 wherein the concentration of the second blocker oligonucleotide is about 5 to about 1,000 times greater than the concentration of the second primer oligonucleotide.

18. The oligonucleotide composition of any of paragraphs 1-17 further comprising a reagent necessary for polymerase chain reaction.

19. The oligonucleotide composition of any of paragraphs 1-17 further comprising a plurality of nucleoside triphosphates.

20. The oligonucleotide composition of any of paragraphs 1-17 further comprising a DNA polymerase.

21. The oligonucleotide composition of any of paragraphs 1-17 further comprising a reagent necessary for polymerase chain reaction, a plurality of nucleoside triphosphates, a DNA polymerase.

22. The oligonucleotide composition of any of paragraphs 1-17 wherein the blocker variable subsequence is a single nucleotide.

23. A method for amplification of a target sequence comprising the steps of:

a. obtaining a sample containing one or more copies of a first nucleic acid comprising a variant sequence and possibly containing at least one copy of a second nucleic acid comprising the target sequence, wherein the target sequence and variant sequence each comprise a homologous subsequence and a variable subsequence, wherein the variable subsequence comprises at least one nucleotide, and wherein the variable subsequence of the target sequence is a target-specific subsequence and the variable subsequence of the variant sequence is a non-target specific subsequence;

b. introducing a blocker oligonucleotide to the sample, wherein the blocker oligonucleotide comprises a first sequence comprising a target-neutral subsequence and a blocker variable subsequence, wherein the target-neutral subsequence is complementary to a portion of the homologous subsequence and the blocker variable subsequence is complementary to the non-target specific subsequence, wherein the blocker variable subsequence is flanked on its 3' and 5' ends by the target-neutral subsequence and is continuous with the target-neutral subsequence; and

c. introducing a first primer oligonucleotide to the sample, wherein the first primer oligonucleotide is sufficient to induce enzymatic extension, wherein the first primer oligonucleotide comprises a second sequence, wherein the second sequence is complementary to a second portion of the homologous subsequence, wherein the second sequence overlaps the target-neutral subsequence by at least 5 nucleotides such that the second sequence comprises an overlapping subsequence and a non-overlapping subsequence, and wherein the second sequence does not include any sequence complementary to the variable subsequence;

d. introducing to the sample a DNA polymerase, nucleoside triphosphates, and one or more reagents necessary for polymerase-based nucleic acid amplification; and

e. reacting the sample under conditions sufficient to achieve nucleic acid amplification.

24. The method of paragraph 23 wherein the blocker oligonucleotide comprises a functional group or a non-complementary sequence region at or near the 3' end, which prevents enzymatic extension.

25. The method of paragraph 24 wherein the functional group comprises a 3-carbon spacer or a dideoxynucleotide.

26. The method of paragraph 23 wherein the DNA polymerase is a thermostable DNA polymerase.

27. The method of paragraph 26 wherein the conditions sufficient to achieve nucleic acid amplification comprise exposing the sample to at least 10 cycles, wherein each cycle comprises at least 2 different temperature exposures, one temperature exposure of at least 85 °C, and one temperature exposure of no more than 75 °C.

28. The method of paragraph 23 further comprising the step of introducing to the sample an enzyme selected from the group consisting of a nicking enzyme, a recombinase, a helicase, a RNAse, a reverse transcriptase, or any combination thereof.

29. The method of paragraph 23 wherein the overlapping subsequence comprises a portion of the 5' end of the target-neutral subsequence, wherein said portion is from about 5 nucleotides to about 40 nucleotides.

30. The method of paragraph 23 wherein the overlapping subsequence comprises a portion of the 5' end of the target-neutral subsequence, wherein said portion is from about 7 nucleotides to about 30 nucleotides.

31. The method of paragraph 23 wherein the second sequence yields a standard free energy of hybridization ($\Delta G°_{PT}$) and the first sequence yields a standard free energy of hybridization ($\Delta G°_{BT}$), which satisfies the following condition:

$$+2 \text{ kcal/mol} \geq \Delta G°_{PT} - \Delta G°_{BT} \geq -8 \text{ kcal/mol}.$$

32. The method of paragraph 23 wherein the non-overlapping subsequence yields a standard free energy of hybridization ($\Delta G^\circ_3$), which satisfies the following condition:

$$-4 \text{ kcal/mol} \geq \Delta G^\circ_3 \geq -12 \text{ kcal/mol}.$$

33. The method of paragraph 23 wherein the concentration of the blocker oligonucleotide introduced into the sample is about 2 to about 10,000 times greater than the concentration of the first primer oligonucleotide introduced into the sample.

34. The method of paragraph 23 wherein the concentration of the blocker oligonucleotide introduced into the sample is about 5 to about 1,000 times greater than the concentration of the first primer oligonucleotide introduced into the sample.

35. The method of paragraph 23 further comprising the step of introducing a second primer oligonucleotide into the sample, wherein the second primer oligonucleotide comprises a third sequence, wherein the third sequence does not overlap with the variable subsequence, and wherein the third sequence is target-neutral and is sufficient to use with the first primer oligonucleotide to amplify a region of a nucleic acid comprising the target sequence.

36. The method of paragraph 35 further comprising the step of introducing a second blocker oligonucleotide to the sample, wherein the second blocker oligonucleotide comprises a fourth sequence comprising a second target-neutral subsequence and a second blocker variable subsequence, wherein the second blocker variable subsequence is the complementary sequence to the blocker variable subsequence, wherein the second blocker variable subsequence is flanked on its 3' and 5' ends by the second target-neutral subsequence and is continuous with the second target-neutral subsequence, wherein the third sequence overlaps the second target-neutral subsequence by at least 5 nucleotides such that the third sequence comprises a second overlapping subsequence and a second non-overlapping subsequence.

37. The method of paragraph 36 wherein the second blocker oligonucleotide comprises a second functional group or a second non-complementary sequence region at or near the 3' end, which prevents enzymatic extension.

38. The method of paragraph 37 wherein the second functional group is selected from the group consisting of a 3-carbon spacer or a dideoxynucleotide.

39. The method of paragraph 36 wherein the second overlapping subsequence comprises a portion of the 5' end of the second target-neutral subsequence, wherein said portion is from about 5 nucleotides to about 40 nucleotides.

40. The method of paragraph 36 wherein the second overlapping subsequence comprises a portion of the 5' end of the second target-neutral subsequence, wherein said portion is from about 7 nucleotides to about 30 nucleotides.

41. The method of paragraph 36 wherein the third sequence yields a standard free energy of hybridization ($\Delta G^\circ_{PT2}$) and the fourth sequence yields a standard free energy of hybridization ($\Delta G^\circ_{BT2}$), which satisfies the following condition:

$$+2 \text{ kcal/mol} \geq \Delta G^\circ_{PT2} - \Delta G^\circ_{BT2} \geq -8 \text{ kcal/mol}.$$

42. The method of paragraph 36 wherein the second non-overlapping subsequence yields a standard free energy of hybridization ($\Delta G^\circ_6$), which satisfies the following condition:

$$-4 \text{ kcal/mol} \geq \Delta G^\circ_3 \geq -12 \text{ kcal/mol}.$$

43. The method of paragraph 36 wherein the concentration of the second blocker oligonucleotide introduced into the sample is about 2 to about 10,000 times greater than the concentration of the second primer oligonucleotide introduced into the sample.

44. The method of paragraph 36 wherein the concentration of the second blocker oligonucleotide introduced into the sample is about 5 to about 1,000 times greater than the concentration of the second primer oligonucleotide introduced into the sample.

45. The method of paragraph 23 following step (e), removing an aliquot from the sample and repeating steps (b) through (e).

It should be understood that various changes and modifications to the presently preferred embodiments described herein will be apparent to those skilled in the art. Such changes and modifications can be made without departing from the spirit and scope of the present invention and without diminishing its intended advantages. It is therefore intended that such changes and modifications be covered by the following claims.

**Claims**

1. A method for amplification of a target sequence comprising the steps of:

a. obtaining a sample containing one or more copies of a first nucleic acid comprising a variant sequence and possibly containing at least one copy of a second nucleic acid comprising the target sequence, wherein the target sequence and variant sequence each comprise a homologous subsequence and a variable subsequence, wherein the variable subsequence comprises an insertion or deletion that is present in the target sequence but not the variant sequence, and wherein the variable subsequence of the target sequence is a target-specific subsequence and the variable subsequence of the variant sequence is a non-target specific subsequence;

b. introducing a blocker oligonucleotide to the sample comprising a first sequence comprising a target-neutral subsequence and a blocker variable subsequence, wherein the blocker variable subsequence is flanked on its 3' and 5' ends by the target-neutral subsequence and is continuous with the target-neutral subsequence, wherein the blocker variable subsequence divides the target-neutral subsequence into two portions, a portion to the 3' of the blocker variable subsequences and a portion to the 5' of the blocker variable subsequence, wherein the target-neutral subsequence is complementary to a portion of the homologous subsequence of both the first target nucleic acid and a first variant nucleic acid, wherein the blocker variable subsequence is complementary to a first variant subsequence of the first variant nucleic acid, and wherein the blocker oligonucleotide comprises a non-complementary sequence region at its 3' end, which prevents enzymatic extension and which is not complementary to a sequence continuous with the target-neutral subsequence; and

c. introducing a first primer oligonucleotide to the sample, wherein the first primer oligonucleotide is sufficient to induce enzymatic extension, wherein the first primer oligonucleotide comprises a second sequence, wherein the second sequence is complementary to a second portion of the homologous subsequence, wherein the second sequence overlaps the target-neutral subsequence of the blocker oligonucleotide by at least 5 nucleotides such that the second sequence comprises an overlapping subsequence and a non-overlapping subsequence, and wherein the second sequence does not include any sequence complementary to the variable subsequence; wherein the second sequence of the first primer oligonucleotide yields a standard free energy of hybridization ($\Delta G°PT$) to the target sequence, and the first sequence of the blocker oligonucleotide yields a standard free energy of hybridization ($\Delta G°BT$) to the target sequence, which satisfies the following condition: $+2 \text{ kcal/mol} \geq \Delta G°PT - \Delta G°BT \geq -8 \text{ kcal/mol}$; and wherein the non-overlapping subsequence yields a standard free energy of hybridization ($\Delta G°3$) to the target sequence, which satisfies the following condition: $-4 \text{ kcal/mol} \geq \Delta G°3 \geq -12 \text{ kcal/mol}$;

d. introducing to the sample a DNA polymerase, nucleoside triphosphates, and one or more reagents necessary for polymerase-based nucleic acid amplification; and

e. reacting the sample under conditions sufficient to achieve nucleic acid amplification.

2. The method of claim 1, wherein the variable subsequence is an insertion that is present in the target sequence but not in the variant sequence, wherein the variable subsequence of the variant sequence is absent, and wherein the blocker variable subsequence is absent.

3. The method of claim 1, wherein the variable subsequence is a deletion that is present in the target sequence but not in the variant sequence.

4. The method of claim 1, wherein the DNA polymerase is a thermostable DNA polymerase, and wherein the conditions sufficient to achieve nucleic acid amplification comprise exposing the sample to at least 10 cycles, wherein each cycle comprises at least 2 different temperature exposures, one temperature exposure of at least 85 °C, and one temperature exposure of no more than 75 °C.

5. The method of claim 1, wherein the overlapping subsequence comprises a portion of the 5' end of the target-neutral subsequence, wherein said portion is from about 5 nucleotides to about 40 nucleotides, or optionally, is from about 7 nucleotides to about 30 nucleotides.

6. The method of claim 1, wherein the concentration of the blocker oligonucleotide introduced into the sample is about 2 to about 10,000 times greater than the concentration of the first primer oligonucleotide introduced into the sample.

7. The method of claim 1, wherein the concentration of the blocker oligonucleotide introduced into the sample is about 5 to about 1,000 times greater than the concentration of the first primer oligonucleotide introduced into the sample.

8. An oligonucleotide composition for carrying out the method of any of claims 1-7, comprising:

(a) a first blocker oligonucleotide comprising , a first sequence comprising a first target-neutral subsequence and a first blocker variable subsequence, wherein the first blocker variable subsequence is flanked on its 3' and 5' ends by the first target-neutral subsequence and is continuous with the first target-neutral subsequence, wherein the first blocker variable subsequence divides the first target-neutral subsequence into two portions, a portion to the 3' of the first blocker variable subsequences and a portion to the 5' of the first blocker variable subsequence, wherein the first target-neutral subsequence is complementary to a first portion of a homologous subsequence of both a first target nucleic acid and a first variant nucleic acid, wherein the first blocker variable subsequence is complementary to a first variant subsequence of the first variant nucleic acid, and wherein the first blocker oligonucleotide comprises a non-complementary sequence region at its 3' end, which prevents enzymatic extension and which is not complementary to a sequence continuous with the first target-neutral subsequence; and

(b) a first primer oligonucleotide sufficient to induce enzymatic extension for amplification of a desired first target sequence, wherein the first primer oligonucleotide comprises a second sequence that is complementary to a second portion of a homologous subsequence of both the first target nucleic acid and the first variant nucleic acid, wherein the second sequence overlaps the target-neutral subsequence by at least 5 nucleotides such that the second sequence comprises a first overlapping subsequence and a first non-overlapping subsequence, and wherein the second sequence does not include the first blocker variable subsequence, wherein the second sequence yields a standard free energy of hybridization ($\Delta G°PT$) to the target nucleic acid, and wherein the first sequence yields a standard free energy of hybridization ($\Delta G°BT$) to the target nucleic acid, which satisfies the following condition: +2 kcal/mol $\geq \Delta G°PT$ - $\Delta G°BT \geq$ -8 kcal/mol; and wherein the first non-overlapping subsequence of the first primer oligonucleotide yields a standard free energy of hybridization ($\Delta G°3$) to the target nucleic acid, which satisfies the following condition: -4 kcal/mol $\geq \Delta G°3 \geq$ -12 kcal/mol;

(c) a second blocker oligonucleotide comprising a fourth sequence comprising a second target-neutral subsequence and a second blocker variable subsequence, wherein the second blocker variable subsequence is flanked on its 3' and 5' ends by the second target-neutral subsequence and is continuous with the second target-neutral subsequence, wherein the second blocker variable subsequence divides the second target-neutral subsequence into two portions, a portion to the 3' of the second blocker variable subsequences and a portion to the 5' of the second blocker variable subsequence, wherein the second target-neutral subsequence is complementary to a first portion of a homologous subsequence of both a second target nucleic acid and a second variant nucleic acid, wherein the second blocker variable subsequence is complementary to a second variant subsequence of the second variant nucleic acid, and wherein the second blocker oligonucleotide comprises a non-complementary sequence region at its 3' end, which prevents enzymatic extension and which is not complementary to a sequence continuous with the second target-neutral subsequence; and

(d) a second primer oligonucleotide sufficient to induce enzymatic extension for amplification of a desired second target sequence, wherein the second primer oligonucleotide comprises a third sequence that is complementary to a second portion of a homologous subsequence of both the second target nucleic acid and the second variant nucleic acid, wherein the third sequence overlaps the second target-neutral subsequence by at least 5 nucleotides such that the third sequence comprises a second overlapping subsequence and a second non-overlapping subsequence, and wherein the third sequence does not include the second blocker variable subsequence,

wherein the third sequence yields a standard free energy of hybridization ($\Delta G°PT2$) to the second target nucleic acid, and wherein the fourth sequence yields a standard free energy of hybridization ($\Delta G°BT2$) to the target nucleic acid, which satisfies the following condition: +2 kcal/mol $\geq \Delta G°PT2$ - $\Delta G°BT2 \geq$ -8 kcal/mol; and wherein the second non-overlapping subsequence of the second primer oligonucleotide yields a standard free energy of hybridization ($\Delta G°3$) to the second target nucleic acid, which satisfies the following condition: -4 kcal/mol $\geq \Delta G°3 \geq$ -12 kcal/mol.

9. The oligonucleotide composition of claim 8, wherein:

(a) the first overlapping subsequence comprises a portion of the 5' end of the first target-neutral subsequence, wherein the portion is from about 5 nucleotides to about 40 nucleotides;
(b) the second overlapping subsequence comprises a portion of the 5' end of the second target-neutral subsequence, wherein the portion is from about 5 nucleotides to about 40 nucleotides; or
(c) both (a) and (b).

10. The oligonucleotide composition of claim 8, wherein:

(a) the first overlapping subsequence comprises a portion of the 5' end of the first target-neutral subsequence, wherein the portion is from about 7 nucleotides to about 30 nucleotides;
(b) the second overlapping subsequence comprises a portion of the 5' end of the second target-neutral subsequence, wherein the portion is from about 7 nucleotides to about 30 nucleotides; or
(c) both (a) and (b).

11. The oligonucleotide composition of claim 8, wherein:

(a) the concentration of the first blocker oligonucleotide is about 2 to about 10,000 times greater than the concentration of the first primer oligonucleotide;
(b) the concentration of the second blocker oligonucleotide is about 2 to about 10,000 times greater than the concentration of the second primer oligonucleotide; or
(c) both (a) and (b).

12. The oligonucleotide composition of claim 8, wherein:

(a) the concentration of the first blocker oligonucleotide is about 5 to about 1,000 times greater than the concentration of the first primer oligonucleotide;
(b) the concentration of the second blocker oligonucleotide is about 5 to about 1,000 times greater than the concentration of the second primer oligonucleotide;
(c) both (a) and (b).

13. The oligonucleotide composition of claim 8, wherein the oligonucleotide composition further comprises:

(a) a reagent necessary for polymerase chain reaction; or
(b) a plurality of nucleoside triphosphates; or
(c) a DNA polymerase.

14. The oligonucleotide composition of claim 8, wherein:

(a) the first blocker variable subsequence is absent;
(b) the second blocker variable subsequence is absent; or
(c) both (a) and (b).

15. The oligonucleotide composition of claim 8, wherein:

(a) the concentration of the first blocker oligonucleotide is about 10 to about 500 times greater than the concentration of the first primer oligonucleotide; and/or
(b) the concentration of the second blocker oligonucleotide is about 10 to about 500 times greater than the concentration of the second primer oligonucleotide.

16. The oligonucleotide composition of claim 8, further comprising a target nucleic acid.

FIG. 1

FIG. 2

Primer 1 2
AGCTGACCTAA
TCGACTGGATTC**G**CTA
5 6 7 Variant

Primer 1 2
AGCTGACCTAA
TCGACTGGATTC**A**CTA
5 6 8 Target

3 4 Blocker
ACCTAAG**C**GAT
TCGACTGGATTC**G**CTA
5 6 7 Variant

3 4 Blocker
ACCTAAG**C**GAT
TCGACTGGATTC**A**CTA
5 6 8 Target

$$\Delta G°_{PV} = \Delta G°_{1\text{-}5} + \Delta G°_{2\text{-}6}$$

$$\Delta G°_{1\text{-}5} = \Delta G°_{init} + \Delta G°\left(\frac{AG}{TC}\right) + \Delta G°\left(\frac{GC}{CG}\right) + \Delta G°\left(\frac{CT}{GA}\right) + \Delta G°\left(\frac{TG}{AC}\right) + \Delta G°\left(\frac{GA}{CT}\right)$$

$$\Delta G°_{2\text{-}6} = \Delta G°\left(\frac{AC}{TG}\right) + \Delta G°\left(\frac{CC}{GG}\right) + \Delta G°\left(\frac{CT}{GA}\right) + \Delta G°\left(\frac{TA}{AT}\right) + \Delta G°\left(\frac{AA}{TT}\right)$$

$$\Delta G°_{PT} = \Delta G°_{1\text{-}5} + \Delta G°_{2\text{-}6} = \Delta G°_{PV}$$

$$\Delta G°_{BV} = \Delta G°_{3\text{-}6} + \Delta G°_{4\text{-}7}$$

$$\Delta G°_{3\text{-}6} = \Delta G°\left(\frac{AC}{TG}\right) + \Delta G°\left(\frac{CC}{GG}\right) + \Delta G°\left(\frac{CT}{GA}\right) + \Delta G°\left(\frac{TA}{AT}\right) + \Delta G°\left(\frac{AA}{TT}\right)$$

$$\Delta G°_{4\text{-}7} = \Delta G°_{init} + \Delta G°\left(\frac{AG}{TC}\right) + \Delta G°\left(\frac{GC}{CG}\right) + \Delta G°\left(\frac{CG}{GC}\right) + \Delta G°\left(\frac{GA}{CT}\right) + \Delta G°\left(\frac{AT}{TA}\right)$$

$$\Delta G°_{BT} = \Delta G°_{3\text{-}6} + \Delta G°_{4\text{-}8}$$

$$\Delta G°_{4\text{-}8} = \Delta G°_{init} + \Delta G°\left(\frac{AG}{TC}\right) + \Delta G°\left(\frac{GC}{CA}\right) + \Delta G°\left(\frac{CG}{AC}\right) + \Delta G°\left(\frac{GA}{CT}\right) + \Delta G°\left(\frac{AT}{TA}\right)$$

FIG. 3

Forward Primer
CAGCCTCATCCAAAAGAGGAAA

$\Delta G^\circ_{rxn1}$ = 3.38 kcal/mol

Blocker AAAAGAGGAAA C AGGACCCCAGAGCTC

Reverse Primer Sequence

. . . . . AGTGTCGGAGTAGGTTTTCTCCTTT A TCCTGGGGTCTCGAGGGA . . . . . . TTACGACCCCAAATCTCACTC . . . . .
Target NA18537

. . . . . AGTGTCGGAGTAGGTTTTCTCCTTT G TCCTGGGGTCTCGAGGGA . . . . . . TTACGACCCCAAATCTCACTC . . . . .
Variant NA18562

SMAD7 rs4939827

FIG. 4

| Extension Temperature (°C) | Target Cq | ΔCq |
|---|---|---|
| 56 | 28.4 | 12.5 |
| 58 | 24.7 | 8.9 |
| 60 | 25.3 | 13.2 |
| 62 | 26.7 | 13.5 |
| 64 | 36.8 | 17.0 |
| 66 | 58.7 | - |

Working Range: 56-64°C

Primer

TGTATATAGACGGTAAAATAAACACCAAGA

Blocker ACACCAAGA^C GTGGTAAATATTTACCTGGTC

. . . . . AACATATATCTGCCATTTTATTTGTGGTTCTC CACCATTTATAAATGGACCAGGGAC . . . . . .
Target                                    NA18562

. . . . . AACATATATCTGCCATTTTATTTGTGGTTCTG CACCATTTATAAATGGACCAGGGAC . . . . . .
Variant                                  NA18537

BRAF rs3789806

## FIG. 5

FIG. 6A

FIG. 6B

Simulation of Effects of $\Delta G^{\circ}_{rxn1}$, Given Fixed $\Delta\Delta G^{\circ} = 2$ kcal/mol

FIG. 7A

Experimental Validation on SMAD7 rs4939827

Primer
CAGCCTCATCCAAAAGAGGAAA⟶

| $\Delta G^{\circ}_{rxn1}$ (kcal/mol) | Target Cq | $\Delta$Cq |
|---|---|---|
| 0.13 | 23.2 | 6.1 |
| 0.73 | 23.3 | 10.9 |
| 2.19 | 23.6 | 12.9 |
| 2.78 | 23.7 | 15.4 |
| 3.38 | 24.8 | 17.4 |
| 4.54 | 25.6 | 14.7 |

Blocker C1  AAAAGAGGAAAᶜAGGACCCCAGA

Blocker C2  AAAAGAGGAAAᶜAGGACCCCAGAG

Blocker C3  AAAAGAGGAAAᶜAGGACCCCAGAGC

Blocker C4  AAAAGAGGAAAᶜAGGACCCCAGAGCT

Blocker C5  AAAAGAGGAAAᶜAGGACCCCAGAGCTC

Blocker C6  AAAAGAGGAAAᶜAGGACCCCAGAGCTCC

.....AGTGTCGGAGTAGGTTTTCTCCTTT[A]TCCTGGGGTCTCGAGGGA.....
Target

.....AGTGTCGGAGTAGGTTTTCTCCTTT[G]TCCTGGGGTCTCGAGGGA.....
Variant

FIG. 7B(I)

Experimental Validation on SMAD7 rs4939827

| | | Primer | | | $\Delta G°_{rxn1}$ (kcal/mol) | Target Cq | $\Delta$Cq |
|---|---|---|---|---|---|---|---|
| | | CAGCCTCATCCAAAAGAGGAAA | | | -2.16 | 23.3 | 1.7 |
| Blocker T1 | | AAAAGAGGAAA$^T$AGGACCCCAGA | | | -1.57 | 23.5 | 6.6 |
| Blocker T2 | | AAAAGAGGAAA$^T$AGGACCCCAGAG | | | -0.11 | 25.8 | 10.1 |
| Blocker T3 | | AAAAGAGGAAA$^T$AGGACCCCAGAGC | | | 0.49 | 27.3 | 9.3 |
| Blocker T4 | | AAAAGAGGAAA$^T$AGGACCCCAGAGCT | | | 1.08 | 29.9 | 8.8 |
| Blocker T5 | | AAAAGAGGAAA$^T$AGGACCCCAGAGCTC | | | 2.24 | 32.6 | 8.8 |
| Blocker T6 | | AAAAGAGGAAA$^T$AGGACCCCAGAGCTCC | | | | | |

.....AGTGTCGGAGTAGGTTTTCTCCTTT⌐G⌐TCCTGGGGTCTCGAGGGA.....
　　　　　Target

.....AGTGTCGGAGTAGGTTTTCTCCTTT⌐A⌐TCCTGGGGTCTCGAGGGA.....
　　　　　Variant

# FIG. 7B(II)

Primer

TGTATATAGACGGTAAAATAAACACCAAGA

Blocker ACACCAAGACGTGGTAAATATTTACCTGGTC AAAA

......AACATATATCTGCCATTTTATTTGTGGTTCTCCACCATTTATAAATGGACCAGGGAC......
Target

......AACATATATCTGCCATTTTATTTGTGGTTCTGCACCATTTATAAATGGACCAGGGAC......
Variant

BRAF rs3789806

FIG. 8

FIG. 9A

FIG. 9B

FIG. 10

Primer

CGATATGGTTTGTTTCAAGGTATGATTTTTA

Blocker-I

AAGGTATGATTTTTAATTAAAAATTGTTGTTCACATTTGAAGG

......TAGCTATACCAAACAAAGTTCCATACTAAAAATTAATTTTT --- AACAAGTGTAAACTTCCA......
Target                                                                    NA18562

......TAGCTATACCAAACAAAGTTCCATACTAAAAATTAATTTTT AAC AACAAGTGTAAACTTCCA......
Variant                                      Detect Deletion      NA18537

Target Cq = 26.3

ΔCq = 16.6

— NA18537
— NA18562

## FIG. 11A

Primer

CGATATGGTTTGTTTCAAGGTATGATTTTTA

Blocker-D ——— AAGGTATGATTTTTAATTAAAAA - - - TTGTTCACATTTGAAGG

......TAGCTATACCAAACAAAGTTCCATACTAAAAATTAATTTTT AAC AACAAGTGTAAACTTCCA......

Target                                                                                    NA18537

......TAGCTATACCAAACAAAGTTCCATACTAAAAATTAATTTTT - - - AACAAGTGTAAACTTCCA......

Variant                                                    Detect Insertion        NA18562

Target Cq = 27.8
ΔCq = 16.3

FIG. 11B

Primer GTGGTAAATATTTACCTGGTCCCTG

Blocker GGTAAAATAAACACCAAGAᴄGTGGTAAATATTTAC

......GCCATTTTATTTGTGGTTCTᴄCACCATTTATAAATGGACCAGGGACA......
Target

......GCCATTTTATTTGTGGTTCTGCACCATTTATAAATGGACCAGGGACA......
Variant       BRAF rs3789806

Target Cq = 23.9

ΔCq = 2.5

Target   Variant

FIG. 12

FIG. 13

Forward Primer
TGGAGCCTTGTATATAGACGGTAAAAT

Forward Blocker
GACGGTAAAATAAACACCAAGAᶜGTGGTAAA

...TGACCTCGGAACATATATCTGCCATTTTATTTGTGGTTCTⒸCACCATTTATAAATGGACCAGGGACAACAACTACAA...⎰ Reverse
Target

...TGACCTCGGAACATATATCTGCCATTTTATTTGTGGTTCTⒼCACCATTTATAAATGGACCAGGGACAACAACTACAA...⎱ Template
Variant                                                    BRAF rs3789806

...ACTGGAGCCTTGTATATAGACGGTAAAATAAACACCAAGAⒸGTGGTAAATATTTACCTGGTCCCTGTTGTTGATGTT...⎰ Forward
Variant

...ACTGGAGCCTTGTATATAGACGGTAAAATAAACACCAAGAⒼGTGGTAAATATTTACCTGGTCCCTGTTGTTGATGTT...⎱ Template
Target
                              TTGTGGTTCTᴳCACCATTTATAAATGGACCA
                              Reverse Blocker

                                        TATAAATGGACCAGGGACAACAACTAC
                                        Reverse Primer

FIG. 14

**REFERENCES CITED IN THE DESCRIPTION**

**Patent documents cited in the description**

- US 62000114
- WO 26094700251 A